(19) Europäisches Patentamt
European Patent Office
Office européen des brevets

(11) EP 2 266 947 A1

(12) **EUROPEAN PATENT APPLICATION**

(43) Date of publication:
**29.12.2010 Bulletin 2010/52**

(21) Application number: **09382098.3**

(22) Date of filing: **18.06.2009**

(51) Int Cl.:
*C07C 205/55* (2006.01) *C07C 69/757* (2006.01)
*C07D 307/46* (2006.01) *C07D 307/80* (2006.01)
*C07D 333/72* (2006.01) *A61K 31/216* (2006.01)
*A61K 31/341* (2006.01) *A61K 31/343* (2006.01)
*A61K 31/381* (2006.01) *A61P 31/06* (2006.01)
*A61P 31/04* (2006.01) *A61P 31/00* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(71) Applicant: **Universidad De Santiago De Compostela**
**15782 Santiago de Compostela (ES)**

(72) Inventors:
- **González Bello, Concepción**
  **15782 Santiago de Compostela (ES)**
- **Sánchez Sixto, Cristina**
  **15782 Santiago de Compostela (ES)**
- **Sedes Diaz, Antia**
  **15782 Santiago de Compostela (ES)**

(74) Representative: **ABG Patentes, S.L.**
**Avenida de Burgos 16D**
**Edificio Euromor**
**28036 Madrid (ES)**

(54) **Ester derivatives as competitive inhibitors of type II dehydroquinase enzyme**

(57) The present invention is directed to a compound of formula I, its diastereoisomers, its enantiomers or its pharmaceutically acceptable salts or solvates, to procedures of obtaining the same, and use as competitive inhibitors of the third enzyme of the shikimic acid pathway, the type 11 dehydroquinase.

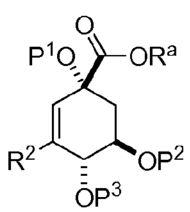

**I**

EP 2 266 947 A1

## Description

### FIELD OF THE INVENTION

The present invention relates to compounds of general formula **I**, to procedures of obtaining the same, and their use as competitive inhibitors of the third enzyme of the shikimic acid pathway, the type II dehydroquinase.

### STATE OF THE ART

Although nowadays enormous effective chemotherapeutic agents have been developed, the number of deaths among hospitalized patients infected with resistant bacterial strains has increased dramatically. This fact is especially remarkable for important diseases such as tuberculosis, where the current therapies become less efficient. Their effects are particularly strong in people with a compromised immune system such as HIV patients. The synergy between the AIDS epidemic and increasing surge of multidrug-resistant isolates to antibiotics leads to the alarming conclusion that antibiotics are loosing their effectiveness. It is therefore necessary to discover new, safe, selective and more efficient antibiotics to face this problem.

For example, selectivity can sometimes be achieved by using compounds that inhibit one of the biosynthetic pathways present in bacteria. Thus, there are antibiotics that interfere in the protein, lipids or catetenoids biosynthesis, etc. In bacteria, there is a metabolic route, known as the shikimic acid pathway (Haslam, E. The shikimate pathway. New York: Wiley; 1974), through which chorismic acid is biosynthesized. The later compound is the precursor in the synthesis of aromatic compounds such as the aromatic amino acids, folates, ubiquinones and certain vitamins (Abell, C. Enzymology and molecular biology of the shikimate pathway. In: Sankawa U, editor. Comprehensive Natural Products Chemistry. Oxford: Pergamon, Elsevier Science Ltd.; 1998. p 573). The shikimic acid pathway is present in bacteria, fungi, higher plants and has recently been discovered in apicomplexan parasites, such as *Cryptosporidium parvum* (Roberts, F.; et all Nature 1998, 393, 801; Roberts, C. W. et all J. Infect. Dis. 2002, 185 (suppl 1), S25; McConkey, G. A.; Pinney, J. W.; Westhead, D. R.; Plueckhahn, K.; Fitzpatrick, T. B.; Macheroux, P.; Kappes, B. Trends in Parasitology 2004, 20, 60).

The enzyme dehydroquinase (3-dehydroquinate dehydratase, EC 4.2.1.10) catalyzes the reversible dehydration of 3-dehydroquinic acid to form 3-dehydroshikimic acid (Scheme 1). There are two different dehydroquinases, known as type I and type II, which possess different biochemical and biophysical properties and do not show sequence similarity (Hawkins, A. R. Curr. Genet. 1987, 11, 491). These two enzymes catalyse the same reaction, but they utilize completely different mechanisms and opposite stereochemistry (Kleanthous, C.; Davis, K.; Kelly, S. M.; Cooper, A.; Harding, S. E.; Price, N. C.; Hawkins, A. R.; Coggins, J. R. Biochem. J. 1992, 282, 687).

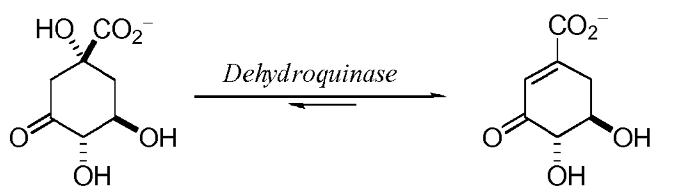


3-dehydroquinic acid 3-dehydroshikimic acid

**Scheme 1**

The type II enzyme (Gourley, D. G.; Coggins, J. R.; Isaacs, N. W.; Moore, J. D.; Charles, I. G.; Hawkins, A. R. J. Mol. Biol. 1994, 241, 488; Krell, T.; Pitt, A. R.; Coggins, J. R. FEBS Lett. 1995, 360, 93), may come from different sources *(Mycobacterium tuberculosis, Streptomyces coelicolor, Helicobacter pylori, Aspergillus nidulans*), and catalyzes the anti elimination of water.

A number of compounds with antibiotic properties have been tested in recent years, some of which are believed to inhibit the dehydroquinase of the shikimic acid pathway. For example, González-Bello, C. et al Org. Biomol. Chem., 1, 2003, p. 2075-2083 or González-Bello, C. et al Medicinal Research Reviews, Vol. 27(2), 2007, p. 177-208 discloses derivative of formula

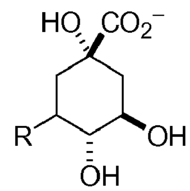

having a $K_i$ of 180 to more than 20,000 micro molar with S. *coelicolor* Type II Dehydroquinase.

González-Bello, C. et al Org. Biomol. Chem., 1, 2003, p. 2075-2083 discloses 3-substituted derivatives of 1,4,5-Trihydroxycyclohexanecarboxylic acid, having the formula

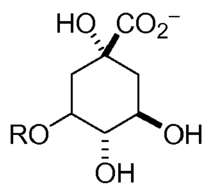

which have a $K_i$ of 180 to more than 20,000 micro molar against S. *coelicolor* Type II Dehydroquinase.

González-Bello, C. et al ChemMedChem, 2008, 3, 756-770 discloses derivative of formula

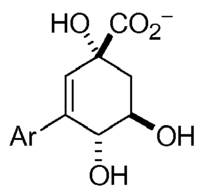

having a $K_i$ of 0.54 to more than 400 micro molar with *H. pylori* Type II Dehydroquinase. Compounds having the above general formula and further compounds where tested in González-Bello, C. et al ChemMedChem, 2007, 2, p. 194-207 against S. *coelicolor* Type II Dehydroquinase, showing a $K_i$ between 0.13 and 33.5 micro molar.

Patent application WO 2005/009330 discloses derivatives of formula

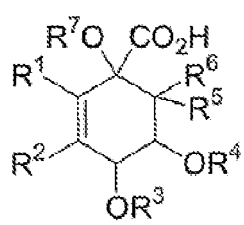

Mainly those wherein $R^3$ and/or $R^7$ are substituted benzyl groups.

The inventors have recently discovered a further family of quinic acid derivatives disclosed in the unpublished European patent application EP08382085.2, which are substituted in position 5 by an alkoxy, thioalkoxy or amine group.

Thus, there is a need to provide further compounds with antibiotic and/or antimicrobial activity.

## SUMMARY OF THE INVENTION

The present invention relates to compounds with antibiotic and/or antimicrobial activity, whose action is based on the effective and selective inhibition of the essential amino acids biosynthesis, particularly, by inhibition of the dehydroquinase, the third enzyme of the shikimic acid pathway.

The present invention provides ester derivatives of the quinic acid structure, which are effective competitive inhibitors of type II dehydroquinase, the third enzyme of the shikimic acid pathway. These esters are significantly more potent than the described compounds of similar structure. The present invention also provides procedures for obtaining these compounds, as well as their use as antibiotics and/or antimicrobials.

Accordingly, a first aspect of the present invention is directed to a compound of formula **I**, its diastereoisomers, its enantiomers or its pharmaceutically acceptable salts or solvates

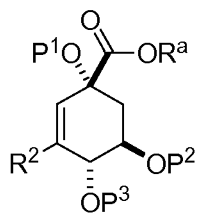

**I**

wherein,

| | |
|---|---|
| $R^a$ | is selected from the group consisting of substituted or unsubstituted alkyl, substituted or unsubstituted alkenyl, substituted or unsubstituted alkynyl, substituted or un- substituted aryl, substituted or unsubstituted heterocyclyl, substituted or unsubs- tituted arylalkyl and substituted or unsubstituted heterocyclylalkyl; |
| each $P^1$, $P^2$ and $P^3$ | is independently selected from the group consisting of hydrogen, substituted or unsubstituted alkyl, substituted or unsubstituted alkenyl, substi- tuted or unsubstituted alkynyl, substituted or unsubstituted silyl, substituted or unsubstituted arylalkyl and -(C=O)$R^a$, wherein $R^a$ is as defined above; and |
| $R^2$ | is selected from the group consisting of substituted or unsubstituted alkyl, substituted or unsubstituted alkenyl, substituted or unsubstituted alkynyl, substituted or un- substituted aryl, substituted or unsubstituted heterocyclyl, substituted or unsubs- tituted arylalkyl and substituted or unsubstituted heterocyclylalkyl. |

Further aspects of the present invention are methods for the synthesis of said compounds of formula **I.**

A further aspect of the present invention is a pharmaceutical composition comprising said compound of formula **I** and a pharmaceutically acceptable carrier.

A further aspect of the present invention is a compound of formula **I** as defined above, for use as a medicament.

A further aspect of the present invention is a compound of formula **I** as defined above, for use as an antibiotic and/or antimicrobial agent.

## DETAILED DESCRIPTION OF THE INVENTION

### Definitions

**"Alkyl"** refers to a straight or branched, cyclic or acyclic hydrocarbon radical consisting of carbon and hydrogen atoms, containing no unsaturation, having 1-12, preferably one to eight, more preferably one to four carbon atoms, and which is attached to the rest of the molecule by a single bond, optionally substituted by one or more substituents selected from the group consisting of an halogen atom, a hydroxyl group, a carboxy group, an alkoxy group, a cyano group, a nitro group, a thioalkoxy group, an heterocyclylalkyl group, an heterocyclyl group or $CF_3$, for example, methyl, ethyl, n-propyl, i-propyl, n-butyl, t-butyl, n-pentyl, cyclopropyl, etc.

**"Alkenyl"** refers to a straight or branched, cyclic or acyclic hydrocarbon radical consisting of carbon and hydrogen atoms, containing at least one unsaturation, conjugated or not, having 2 to 12, preferably two to eight, more preferably two to four carbon atoms, and which is attached to the rest of the molecule by a single bond. Alkenyl radicals may be optionally substituted by one or more substituents such as a halogen atom, hydroxyl group, a carboxy group, an alkoxy group, a cyano group, a nitro group, a thioalkoxy group, an heterocyclylalkyl group, an heterocyclyl group or $CF_3$, such as vinyl, allyl, butenyl (e.g. 1-butenyl, 2-butenyl, 3-butenyl), or pentenyl (e.g. 1-pentenyl, 2-pentenyl, 3-pentenyl, 4-pentenyl).

**"alkynyl"** refers to a straight or branched, cyclic or acyclic hydrocarbon radical consisting of carbon and hydrogen atoms, containing at least one carbon-carbon triple bond, conjugated of not, having two to twelve, preferably two to eight, more preferably two to four carbon atoms, and which is attached to the rest of the molecule by a single bond, such as -CCH, $-CH_2CCH$, $-CCCH_3$, $-CH_2CCCH_3$. Alkynyl radicals may be optionally substituted by one or more substituents such as a halogen atom, hydroxyl group, a carboxy group, an alkoxy group, a cyano group, a nitro group, a thioalkoxy group, an heterocyclylalkyl group, an heterocyclyl group or $CF_3$.

**"Aryl"** refers to an aromatic hydrocarbon with 6 to 10 carbon atoms, such as phenyl or naphtyl, optionally substituted by one or more substituents selected from the group consisting of a halogen atom, hydroxyl group, a carboxy group, an alkoxy group, a cyano group, a nitro group, an thioalkoxy group, an alkyl group or $CF_3$.

**"Silyl"** refers to trialkylsilyl species which are commonly used in organic chemistry as protecting groups, such as those disclosed in Greene, T. W.; Wuts, P. G. M. "Protective Groups in Organic Synthesis", 3˚ Ed., Wiley-Interscience, New York, 1999. According to a particular embodiment, a radical of formula $-SiR^dR^eR^f$ wherein $R^d$, $R^e$ and $R^f$ are independently selected from a substituted or unsubstituted alkyl group or a substituted or unsubstituted aryl group, for example, methyl, ethyl, t-butyl, i-propyl, phenyl, etc.

**"arylalkyl"** refers to a one or various aryl groups bonded to the rest of the molecule by an alkyl radical, for example, benzyl, 3-(phenyl)-propyl, etc.

**"Heterocyclyl"** refers to a stable 3 to 15 membered-ring constituted by carbon atoms and 1 to 5 heteroatoms selected from nitrogen, oxygen and sulphur, preferably a 4 to 8 membered-ring constituted by one or more heteroatoms, and more preferably a 5 to 6 membered-ring with one or more heretoatoms. For the purposes of this invention, heterocyclyl groups can be a monocyclic, bicyclic or tricyclic systems, that can include fused rings; and the nitrogen or sulphur atom in the heterocyclic ring can be optionally oxidized; the nitrogen atom can be optionally quatemarized; and the heterocyclyl radical can be partially or totally saturated. The heterocyclyl radicals can be aromatic (e.g. by having one or more aromatic rings), in which case they are considered as **"heteroaryl"** for the purposes of the present invention. The heterocyclic ring can be substituted by one or more substituents selected from the group consisting of a halogen atom, hydroxyl group, a carboxy group, an alkoxy group, an alkyl group, a thioalkoxy group, a cyano group, a nitro group or $CF_3$. Examples of such heterocycles include, for example, furan, thiophene, pyrrole, imidazole, triazole, isothiazole, benzothiophene, benzofurane, indol, benzoimidazole, tetrahydrofuran.

**"Alkoxy"** refers to a radical of formula -O-alkyl, for example, methoxy, ethoxy, propoxy, etc.

**"Thioalkoxy"** refers to a radical of formula -S-alkyl, for example, thiomethoxy, thioethoxy, thiopropoxy, etc.

**"Amino"** refers to a radical of formula $-NR^bR^c$ wherein each of $R^b$ and $R^c$ is independently selected from the group consisting of hydrogen, substituted or unsubstituted alkyl, substituted or unsubstituted alkenyl, substituted or unsubstituted alkynyl, substituted or unsubstituted aryl, substituted or unsubstituted heterocyclyl, substituted or unsubstituted arylalkyl and substituted or unsubstituted heterocyclylalkyl; or $R^b$ and $R^c$ together form a 5 or 6 membered heterocyclyc ring together with the nitrogen atom to which they are attached.

**"Alkoxycarbonyl"** refers to a radical of formula -C(=O)-O-alkyl.

**"Aminocarbonyl"** refers to a radical of formula $-C(=O)-NR^bR^c$, wherein $R^b$ and $R^c$ are as defined above.

**"Alkylcarbonyl"** refers to a radical of formula -C(=O)-alkyl.

**"Heterocyclylalkyl"** refers to a one or various heterocyclyl groups bonded to the rest of the molecule by an alkyl radical, for example, 2-(thienyl)ethyl, benzothiophenylmethyl, etc.

**"Quinic** acid" refers to (1*S*,3*R*,4*S*,5*R*)-1,3,4,5-tetrahydroxycyclohexanecarboxylic acid. Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a $^{13}C$- or $^{14}C$-enriched carbon or $^{15}N$-enriched nitrogen are within the scope of this invention.

Further, the term **"pharmaceutically acceptable"** refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. According to a particular embodiment ethyl ether, ethyl acetate, ethanol, isopropanol or acetonitrile are used as solvents. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts,

and organic alkali salts such as, for example, ethylenediamine, ethanolamine, N,N-dialkylenethanolamine, triethanolamine, glucamine and basic amino acids salts.

The compounds of the invention may be in crystalline form either as free compounds or as solvates (e.g. hydrates) and it is intended that both forms are within the scope of the present invention. Methods of solvation are generally known within the art. Suitable solvates are pharmaceutically acceptable solvates. In a particular embodiment the solvate is a hydrate.

The compounds of the present invention may include diastereoisomers and/or enantiomers depending on the presence of chiral centres or isomers depending on the presence of multiple bonds (e.g. Z, E). The single isomers, diastereoisomers, enantiomers and mixtures thereof fall within the scope of the present invention.

## Compounds of formula I

According to a particular embodiment, $R^2$ is selected from the group consisting of substituted or unsubstituted aryl, substituted or unsubstituted heterocyclyl, substituted or unsubstituted arylalkyl and substituted or unsubstituted heterocyclylalkyl.

In particular embodiment $R^2$ is a substituted or unsubstituted aryl or arylalkyl, preferably substituted or unsubstituted phenyl, phenylalkyl, phenylalkenyl or napthyl. In a particular embodiment, said aryl is substituted with at least one halogen, preferably fluor or bromo, $CF_3$, nitro, cyano, hydroxyl, or carboxy.

According to a particular embodiment, $R^2$ is a group of formula **II**

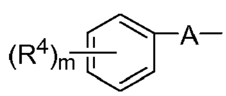

**II**

wherein

A is a bond, or is selected from a substituted or unsubstituted alkyl chain, a substituted or unsubstituted alkenyl chain, or a substituted or unsubstituted alkynyl chain, preferably comprising 1, 2, 3 or 4 chain carbon atoms; m is 0, 1, 2, 3, 4 or 5, preferably 1 or 2; and

$R^4$ is selected from the group consisting of halogen, substituted or unsubstituted alkyl, substituted or unsubstituted alkenyl, substituted or unsubstituted alkynyl, substituted or unsubstituted alkoxy, substituted or unsubstituted thioalkoxy, substituted or unsubstituted aryl, substituted or unsubstituted heterocyclyl, substituted or unsubstituted arylalkyl and substituted or unsubstituted heterocyclylalkyl; wherein $R^4$ may be in any of the free positions.

According to a particular embodiment, $R^4$ is selected from the group consisting of halogen, $CF_3$, nitro, cyano, hydroxyl, and carboxy.

In particular embodiment $R^2$ is a substituted or unsubstituted alkyl, substituted or unsubstituted heteroaryl or substituted or unsubstituted heteroarylalkyl. In a particular embodiment, said heteroaryl or heteroarylalkyl is substituted with at least one halogen, preferably fluor or bromo, $CF_3$, nitro, cyano, hydroxyl, or carboxy.

According to a particular embodiment, $R^2$ is a group of formula **III**

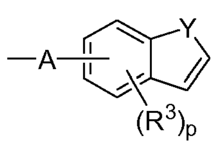

**III**

wherein

A is a bond, or is selected from a substituted or unsubstituted alkyl chain, a substituted or unsubstituted alkenyl chain, or a substituted or unsubstituted alkynyl chain, preferably comprising 1, 2, 3 or 4 chain carbon atoms;

p is 0, 1, 2, or 3, preferably 0 or 1;

$R^3$ is selected from the group consisting of hydrogen, substituted or unsubstituted alkyl, substituted or unsubstituted alkenyl, substituted or unsubstituted alkynyl, substituted or unsubstituted alkoxy, substituted or unsubstituted thioalkoxy, substituted or unsubstituted aryl, substituted or unsubstituted heterocyclyl, substituted or unsubstituted arylalkyl and substituted or unsubstituted heterocyclylalkyl; and

Y is selected from the group consisting of O, S, $NR^b$ and $\oplus NR^bR^c$, wherein $R^b$ and $R^c$ are as defined above,
wherein A and $R^3$ may be in any of the free positions.

According to a particular embodiment, $R^2$ is a group of formula **IV**

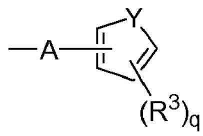

**IV**

wherein
A, Y and $R^3$ are as defined above; and
q is 0, 1, 2, or 3, preferably 1;
wherein A and $R^3$ may be in any of the free positions.

According to a particular embodiment, $R^3$ is substituted or unsubstituted alkyl, halogen, $CF_3$, nitro, cyano, hydroxyl, or carboxy.

According to a particular embodiment, Y is S or O.

According to a particular embodiment, $R^2$ is selected from the group consisting of thiophenyl, furanyl, pyridyl, 1H-[1,2,3]Triazolyl, benzofuranyl, Benzo[b]thiophenyl, 1H-indolyl and benzo[1,2,5]oxadiazole.

According to a particular embodiment, $R^a$ is an alkyl or an alkenyl group, preferably a $C_{1-4}$ alkyl group or a $C_{1-4}$ alkenyl group.

According to a particular embodiment, at least one of $P^1$, $P^2$ and $P^3$ is hydrogen, preferably $P^1$, $P^2$ and $P^3$ are all hydrogen. According to a particular embodiment, at least one of $P^1$, $P^2$ and $P^3$ is a -(C=O)$R^a$ group, wherein $R^a$ is a $C_{1-4}$ alkyl group.

According to a preferred embodiment, the compound of formula **I** is selected from the group consisting of:

isopropyl (1R, 4R, 5R)-1,4,5-trihydroxy-3(3-phenylpropyl)cyclohex-2-en-1-carboxylate;

ethyl (1R, 4R, 5R)-1,4,5-trihydroxy-3(3-phenylpropyl)cyclohex-2-en-1-carboxylate;

methyl (1R, 4R, 5R)-1,4,5-trihydroxy-3(3-phenylpropyl)cyclohex-2-en-1-carboxylate;

methyl (1R, 4R, 5R)-1,4,5-trihydroxy-3-(naphthalen-2-yl)cyclohex-2-en-1-carboxylate;

methyl (1R, 4R, 5R)-3-(benzofuran-5-yl)-1,4,5-trihydroxycyclohex-2-en-1-carboxylate;

methyl (1R, 4R, 5R)-3-(furan-2-yl)-1,4,5-trihydroxycyclohex-2-en-1-carboxylate;

methyl (1R, 4R, 5R)-3-(4-fluorophenyl)-1,4,5-trihydroxycyclohex-2-en-1-carboxylate;

methyl (1R, 4R, 5R)-1,4,5-trihydroxy-3-(3-nitrophenyl)cyclohex-2-en-1-carboxylate;

methyl (1R, 4R, 5R)-3-(benzo[b]thiophen-5-yl)-1,4,5-trihydroxycyclohex-2-en-1-carboxylate;

methyl (1R, 4R, 5R)-1,4,5-trihydroxy-3-(thien-2-yl)cyclohex-2-en-1-carboxylate;

ethyl (1R, 4R, 5R)-1,4,5-triacetoxy-3(3-phenylpropyl)cyclohex-2-en-1-carboxylate;

methyl (1R, 4R, 5R)-1,4,5-triacetoxy-3(3-phenylpropyl)cyclohex-2-en-1-carboxylate;

isopropyl (1R, 4R, 5R)-1,4,5-triacetoxy-3(3-phenylpropyl)cyclohex-2-en-1-carboxylate;

methyl (1R, 4R, 5R)-1,4,5-tributyroxy-3(3-phenylpropyl)cyclohex-2-en-1-carboxylate;

methyl (1R, 4R, 5R)-1,4,5-triacetoxy-3-(naphthalen-2-yl)cyclohex-2-en-1-carboxylate;

methyl (1R, 4R, 5R)-1,4,5-triacetoxy-3-(benzofuran-5-yl)cyclohex-2-en-1-carboxylate;

methyl (1R, 4R, 5R)-3-(benzofuran-5-yl)-1,4,5-tributyroxycyclohex-2-en-1-carboxylate;

methyl (1R, 4R, 5R)-1,4,5-triacetoxy-3-(furan-2-yl)cyclohex-2-en-1-carboxylate;

methyl (1R, 4R, 5R)-1,4,5-triburyroxy-3-(furan-2-yl)cyclohex-2-en-1-carboxylate;

methyl (1R, 4R, 5R)-1,4,5-triacetoxy-3-(benzo[b]thiophen-5-yl)cyclohex-2-en-1-carboxylate;

methyl (1R, 4R, 5R)-3-(benzo[b]thiophen-5-yl)-1,4,5-tributyroxycyclohex-2-en-1-carboxylate;

methyl (1R, 4R, 5R)-1,4,5-triacetoxy-3-(thien-2-yl)cyclohex-2-en-1-carboxylate;

methyl (1R, 4R, 5R)-1,4,5-tributyroxy-3-(naphthalen-2-yl)cyclohex-2-en-1-carboxylate;

methyl (1R, 4R, 5R)-3-(naphthalen-2-yl)-1,4,5-tripentyroxycyclohex-2-en-1-carboxylate;

methyl (1R, 4R, 5R)-1,4,5-tributyroxy-3-(thien-2-yl)cyclohex-2-en-1-carboxylate;

methyl (1R, 4R, 5R)-1,4,5-triacetoxy-3-(3-nitrophenyl)cyclohex-2-en-1-carboxylate;

methyl (1R, 4R, 5R)-1,4,5-tributyroxy-3-(3-nitrophenyl)cyclohex-2-en-1-carboxylate; and

methyl (1R, 4R, 5R)-1,4,5-triacetoxy-3-(4-fluorophenyl)-cyclohex-2-en-1-carboxylate;
its diastereoisomers, its enantiomers or its pharmaceutically acceptable salts or solvates.

**Synthesis of compounds of formula I**

Another aspect of the invention relates to a procedure of obtaining compounds of formula **I**, comprising the ring opening of a lactone of formula **V** in the presence of an alcohol of formula $HOR^a$ and a base wherein $R^a$ is as defined above,

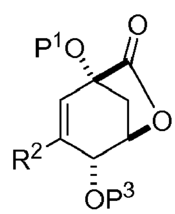

**V**

wherein $P^1$, $P^3$, and $R^2$ are as defined above.

General methods for the ring opening of lactones to yield esters can be used, such as those described in pages 486-7 of March, J. "Advanced Organic Chemistry; Reactions Mechanism and Structure", Wiley-Interscience, fifth ed. According to a preferred embodiment, the reaction involves the use of an alkoxide of formula $R^aO$-, in the corresponding alcohol. Alternative conditions include the reaction in the presence of the alcohol of formula $HOR^a$ and KCN, or in the presence of the alcohol of formula $HOR^a$ and a Lewis acid.

Another aspect of the invention is the synthesis compounds of formula **I,** comprising the esterification of an acid of formula **VI,** preferably in the presence of an alcohol of formula $HOR^a$, wherein $R^a$ is as defined above,

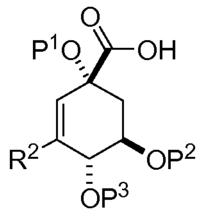

**VI**

wherein $P^1$, $P^2$, $P^3$, and $R^2$ are as defined avobe.

General methods for the esterification of the carboxylic acids of formula VI to yield esters can be used, such as those described in pages 484-6 of March, J. "Advanced Organic Chemistry; Reactions Mechanism and Structure", Wiley-Interscience, fifth ed. The conditions described therein include the use of coupling agents such as DCC, DIC or DHU. Reactions of the compounds of formula VI with diazo compounds, such as diazomethane) is also included in the scope of the invention (see page 490 of March, J. "Advanced Organic Chemistry; Reactions Mechanism and Structure", Wiley-Interscience, fifth ed.).

The compounds of formula **V** and **VI** are known in the art and can be obtained by methods described in, for example, González-Bello, C. et al ChemMedChem., Vol. 2, 2007, p. 194-207 or González-Bello, C. et al ChemMedChem., Vol. 3, 2008, p. 756-770, which contains methods for obtaining compounds of formula **V,** and their subsequent transformation in compounds of formula **VI.** Methods for the preparation of the compounds of the invention can also be found in J. Med. Chem. 2005, 48, 4871-4881.

Also, a lactone of formula **V** can be prepared, subsequently opened so as to obtain the acid of formula **VI** (following the methods described in the previous paragraph), followed by esterification to obtain the compounds of formula **I.**

The scope of the present invention also includes other transformations, usually functional group transformations, which transform a compound of a given formula into a different compound of the same formula. All such transformations are within the scope of the present invention.

For example, a compound of formula **I** wherein $R^2$ comprises an alkenyl group, may be transformed into compound of formula **I** wherein $R^2$ comprises an alkyl group, by performing a catalytic hydrogenation. Alternatively, the catalytic hydrogenation may be performed over the precursor of formula **V** or **VI.** Catalytic hydrogenation can be carried out following known procedures (March, J. "Advanced Organic Chemistry; Reactions Mechanism and Structure", Wiley-Interscience, fifth ed. p. 1002-7), e.g. in the presence of catalyst such as palladium on carbon, palladium hydroxide, Raney Nickel, platinum, ruthenium, platinium oxide or zinc oxide.

In summary, compounds of formula **I** can be obtained by opening of a lactone of formula V or by esterifying an acid of formula **VI.** The ring opening of the lactone can be carried out either in acidic medium, for example, in the presence of an organic acid, such as trifluoroacetic acid, *p*-toluensulfonic acid, camphorsulfonic acid, acetic acid, acidic ion-exchange resin; a Lewis acid or mixtures thereof. The reactions can also be carried our in basic medium, for example, in the presence of an inorganic base, such as $K_2CO_3$, $Na_2CO_3$, or KCN; an organic base, such as a primary amine, a secondary amine, MeONa or EtONa.

Also, any of the compounds of formula **I, V** and **VI** may undergo protection-deprotection reactions using well-known procedures (Greene, T. W.; Wuts, P. G. M. "Protective Groups in Organic Synthesis", 3˚ Ed., Wiley-Interscience, New York, 1999). For example, if $P^1$, $P^2$ and/or $P^3$ is/are a TBS group, the deprotection will be preferably carried out by treatment with tetrabutylamonium fluoride. If $P^1$, $P^2$ and/or $P^3$ is/are a benzyl group, the deprotection will be preferably performed by catalytic hydrogenolysis. If $P^1$, $P^2$ and/or $P^3$ is/are an acetyl group, the deprotection will be preferably carried out by treatment with $K_2CO_3$ in combination with methanol. If $P^1$, $P^2$ and/or $P^3$ is/are methoxyethoxymethyl ether (MEM), the deprotection will be preferably preformed by treatment with trifluoroacetic acid. Also, the hydroxyl groups of the ring can be esterified in order to yield $P^1$, $P^2$ and/or $P^3$ which are a -(C=O)$R^a$ group. Such esterification can be effected under standard conditions such as reaction with the corresponding acyl halide or in the presence of the corresponding anhydride and pyridine (see pages 482-3 of March, J. "Advanced Organic Chemistry; Reactions Mechanism and Structure", Wiley-Interscience, fifth ed.)

## Biological Activity

The compounds of formula **I** are potent competitive inhibitors of type II dehydroquinases from various bacterial sources. This enzyme acts in an essential biosynthetic route in bacteria, the shikimic acid pathway. These compounds have, in many cases, inhibition constants in the low nanomolar range and to even picomolar, which makes them, the

most potent known inhibitors against any dehydroquinase.

Thus a further aspect of the invention is a pharmaceutical composition comprising a compound of formula **I** as defined above and a pharmaceutically acceptable carrier.

A further aspect of the invention is a compound of formula **I** as defined above for use as a medicament.

A further aspect of the invention is the use of a compound of formula **I** for the preparation of a medicament to treat tuberculosis, stomach cancer, gastritis, stomach ulcers, duodenal ulcers, or heartburn. That is, a compound of formula **I** for use in the treatment or prophylaxis of a disease selected from the group consisting of tuberculosis, stomach cancer, gastritis, stomach ulcers, and duodenal ulcers heartburn.

Examples of pharmaceutical compositions include any solid (tablets, pills, capsules, granules etc.) or liquid (solutions, suspensions or emulsions) compositions. Typical administration routes are oral, topical or parenteral administration. In a particular embodiment the pharmaceutical compositions are in oral form. Suitable dose forms for oral administration may be tablets and capsules and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

Suitable pharmaceutical carriers are described in "Remington's Pharmaceutical Sciences" by E.W. Martin

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of many of the diseases to be treated. Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

## EXAMPLES

The Examples, which are detailed next, will have to be considered to better understanding of the present invention, which should not be interpreted as a limitation.

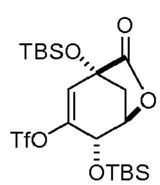

*Preparation of (1R, 4S, 5R)-1,4-di(tert-butyldimethylsilyloxy)cyclohex-2-en-1,5-carbolactone trifluorom ethane sulphonate (A).* To a solution of potassium bis(trimethylsilyl)amide (0.5M in toluene) (3 mL, 1.5 mmol) in dry DMF (1.7 mL), under inert atmosphere at -78 ˚C, a solution of (1*S*, 4*S*, 5*R*)-1,4-di(*tert*-butyldimethylsilyloxy)-3-oxocyclohexan-1,5-carbolactone (500 mg, 1.25 mmol) in dry DMF (2.5 mL) and toluene (1.7 mL) was added dropwise. The resultant mixture was stirred for 1 h at this temperature and a solution of 2-[*N*,*N* bis(trifluoromethylsulfonyl)amino]-5-chloropyridine (735 mg, 1.87 mmol) in dry DMF (1.9 mL) was then added dropwise. The resultant reaction mixture was stirred for 15 h during which period was allowed to warm until room temperature. Water and diethyl ether were added,

the organic layer was separated, and the aqueous layer was extracted twice with diethyl ether. All the combined organic layers were dried (anh. $Na_2SO_4$), filtered and evaporated. The obtained light yellow oil was purified by flash chromatography (eluent: 50% DCM-hexanes) to afford (*1R, 4S, 5R)-1,4-di(tert-butyldimethylsilyloxy)cyclohex-2-en-1,5-carbolactone trifluoromethane sulphonate* ***A*** (665 mg, 80%) as a colourless oil which solidifies on standing. $[\alpha]^{20}$ D -32˚ (c0.6, in $CHCl_3$). $^1H$ NMR (250 MHz, $CDCl_3$) δ 6.05 (s, 1H, H-2), 4.45 (dd, 1H, J= 6.6 and 3.7 Hz, H-5), 4.20 (d, 1H, *J* = 3.7 Hz, H-4), 2.24 (m, 2H, $CH_2$), 0.71 (s, 9H, $C(CH_3)_3$), 0.71 (s, 9H, $C(CH_3)_3$), -0.03 (s, 3H, $SiCH_3$), -0.05 (s, 3H, $SiCH_3$), -0.07 (s, 3H, $SiCH_3$) and -0.07 (s, 3H, $SiCH_3$) ppm. $^{13}C$ NMR (63 MHz, $CDCl_3$) δ 173.7 (C), 146.1 (C), 126.1 (CH), 118.3 (C, $J_{CF}$ 318), 74.5 (CH), 73.6 (C), 67.3 (CH), 36.8 ($CH_2$), 25.5 ($C(CH_3)_3$), 25.4 ($C(CH_3)_3$), 17.9 ($\mathit{C}(CH_3)_3$), 17.8 ($\mathit{C}(CH_3)_3$), -3.3 (2x$SiCH_3$), -5.1 ($SiCH_3$) and -5.1 ($SiCH_3$) ppm. $^{19}F$ NMR (282 MHz, $CDCl_3$) δ -73.7 (s, 3F, $CF_3$) ppm. IR (film): 1814 (C=O) $cm^{-1}$. MS (CI) *m*/*z* (%) 475 ($MH^+$-$CH(CH_3)_3$). HRMS calcd for $C_{16}H_{26}O_7F_3Si_2S$ ($MH^+$-$CH(CH_3)_3$): 475.0890; found: 475.0895.

*General carbon-carbon cross coupling method for Examples 1-4:*

*Procedure A:* To a stirred solution of (*1R*, *4S, 5R*)-1,4-di(*tert*-butyldimethylsilyloxy)cyclohex-2-en-1,5-carbolactone trifluoromethane sulphonate **(A)** (1 equivalent) and $(dba)_3Pd_2.CHCl_3$ (0.075 equivalents) in dry dichloromethane, under inert atmosphere at -78 ˚C, 2 equivalents of a solution of a boronic acid in dry THF (0.15 M) and 3 equivalents of dry triethylamine were added. The dry ice bath was removed, and after 1 h at room temperature more boronic acid (1-1.5 equivalents) in dry THF was added to the reaction mixture cooled at -78 ˚C. The reaction mixture was stirred at room temperature between 4-16 h. The solvents were removed under reduced pressure and the crude residue was purified by flash chromatography.

*Procedure B:* To a stirred solution of (*1R, 4S, 5R*)-1,4-di(*tert*-butyldimethylsilyloxy)cyclohex-2-en-1,5-carbolactone trifluoromethane sulphonate **(A)** (1 equivalent), an ester of a boronic acid (1.1-2.2 equivalents), $Pd(PPh_3)_4$ (0.025 equivalentes), and an aqueous solution of $K_3PO_4$ (1.5 equivalents, 0.8 M) in dioxane was heated at 80-85 ˚C for 2-11 h. After cooling to room temperature, the reaction mixture was diluted with dichloromethane and water. The organic phase was separated and the aqueous layer was extracted with dichloromethane (x2). All the combined organic extracts were dried (anh. $Na_2SO_4$), filtered and concentrated under reduced pressure. The obtained residue was purified by flash chromatography.

*Procedure C*: To a stirred solution of (*1R*, *4S*, *5R*)-1,4-di(*tert*-butyldimethylsilyloxy)cyclohex-2-en-1,5-carbolactone trifluoromethane sulphonate **(A)** (1 equivalent), $PdCl_2(dppf).CH_2Cl_2$ (0.075 equivalents), a boronic acid (1.1 equivalents) and an aqueous solution of $K_2CO_3$ (2.7 equivalents, 1.1 M) in THF (0.1 M) was heated under reflux for 10-15 h. After cooling to room temperature, the reaction mixture was diluted with dichloromethane and water. The organic phase was separated and the aqueous layer was extracted with dichloromethane (x2). All the combined organic extracts were dried (anh. $Na_2SO_4$), filtered and concentrated under reduced pressure. The obtained residue was purified by flash chromatography.

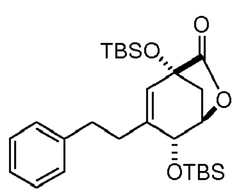

*<u>Example 1:</u> (1R,4R,5R)-1,4-di(butyldimethylsilyloxy)-3-(2-phenylethyl)cyclohex-2-en-1,5- carbolactone* ***(B).*** The cross-coupling method C was employed by using *(1R, 4S, 5R)*-1,4-di(tert-butyldimethylsilyloxy)cyclohex-2-en-1,5-carbolactone trifluoromethane sulphonate **A** as starting material (300 mg, 0.56 mmol), $PdCl_2(dppf).CH_2Cl_2$ (37 mg, 0.04 mmol), 2-phenylethylboronic acid (93 mg, 0.62 mmol), THF (6 mL) and 1.4 mL of $K_2CO_3$. Reaction time: 10 h. Eluent for chromatography: (50%) dicloromethane/hexane. Yield: 220 mg (80%). Colourless oil. $^1H$ NMR (250 MHz, $CDCl_3$) δ 7.39-7.18 (m, 5H, 5xArH), 5.76 (s, 1H, H-2), 4.54 (dd, 1H, *J* = 6.2 and 3.1 Hz, H-5), 4.10 (d, 1H, *J* = 3.1 Hz, H-4), 2.80 (m, 3H, $CH_2$+C*H*H), 2.41 (m, 3H, $CH_2$+CH*H*), 0.99 (s, 9H, $C(CH_3)_3$), 0.98 (s, 9H, $C(CH_3)_3$), 0.22 (s, 6H, 2x$CH_3$), 0.21 (s, 3H, $CH_3$) and 0.18 (s, 3H, $CH_3$) ppm. $^{13}C$ NMR (63 MHz, $CDCl_3$) δ 175.9 (C), 141.0 (C), 138.4 (C), 136.9 (CH), 131.3 (CH), 128.4 (CH), 128.2 (CH), 127.3 (CH), 126.0 (CH), 75.9 (CH), 74.7 (C), 67.8 (CH), 37.2 ($CH_2$), 33.7 ($CH_2$), 33.5 ($CH_2$), 25.7 ($C(\mathit{C}H_3)_3$), 25.6 ($C(\mathit{C}H_3)_3$), 18.0 ($\mathit{C}(CH_3)_3$), 17.9 ($\mathit{C}(CH_3)_3$), -3.1 (2x$SiCH_3$), -4.6 ($SiCH_3$) and -4.6 ($SiCH_3$) ppm. IR (film) 1802 (C=O) $cm^{-1}$.

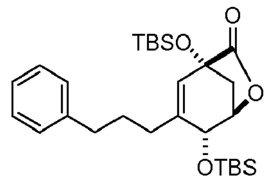

<u>*Example 2:*</u> *(1R,4R,5R)-1,4-di(butyldimethylsilyloxy)-3-(3-phenylpropyl)cyclohex-2-en-1,5- carbolactone* ***(C).*** The cross-coupling method C was employed by using *(1R, 4S, 5R)*-1,4-di(tert-butyldimethylsilyloxy)cyclohex-2-en-1,5-carbolactone trifluoromethane sulphonate **A** as starting material (50 mg, 0.09 mmol), $PdCl_2(dppf).CH_2Cl_2$ (6 mg, 8 μmol), 3-phenylpropylboronic acid (17 mg, 0.10 mmol), THF (1 mL) and 0.2 mL of $K_2CO_3$. Reaction time: 15 h. Eluent for chromatography: (35%) dicloromethane/hexane. Yield: 36 mg (76%). Orange oil. $^1H$ NMR (250 MHz, $CDCl_3$) δ 7.24 (m, 2H, 2xArH), 7.13 (m, 3H, 3xArH), 5.68 (s, 1H, H-2), 4.41 (dd, 1H, J= 6.2 and 3.2 Hz, H-5), 3.96 (d, 1H, *J* = 3.2 Hz, H-4), 2.57 (t, 2H, *J* = 7.5, $CH_2$), 2.27 (m, 2H, $CH_2$), 2.01 (m, 2H, $CH_2$), 1.71 (m, 2H, $CH_2$), 0.89 (s, 9H, $3xCH_3$), 0.85 (s, 9H, $3xCH_3$), 0.15 (s, 3H, $CH_3$), 0.11 (s, 3H, $CH_3$), 0.07 (s, 3H, $CH_3$) and 0.04 (s, 3H, $CH_3$) ppm. $^{13}C$ NMR (63 MHz, $CDCl_3$) δ 176.1 (C), 141.7 (C), 138.9 (C), 130.6 (CH), 128.4 (2xCH), 128.3 (2xCH), 125.9 (CH), 75.9 (CH), 74.7 (C), 67.7 (CH), 37.2 ($CH_2$), 35.5 ($CH_2$), 31.4 ($CH_2$), 28.7 ($CH_2$), 25.6 (2x(C($CH_3$)$_3$), 18.0 (*C*($CH_3$)$_3$), 17.9 (*C*($CH_3$)$_3$), 3.0 ($2xSiCH_3$), 4.6 ($SiCH_3$) and -4.8 ($SiCH_3$) ppm. IR (film): 1802 (C=O) $cm^{-1}$. MS (Cl) *m/z* (%) 503 ($MH^+$). HRMS calcd for $C_{28}H_{47}O_4Si_2$ ($MH^+$): 503.3013; found: 503.3004.

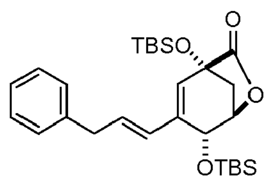

<u>*Example 3:*</u> *(1R,4R,5R)-1,4-di(butyldimethylsilyloxy)-3-(3-phenylprop-1-enyl)cyclohex-2-en-1,5- carbolactone* ***(D).*** The cross-coupling method A was employed by using *(1R, 4S, 5R)*-1,4-di(tert-butyldimethylsilyloxy)cyclohex-2-en-1,5-carbolactone trifluoromethane **sulphonate A** as starting material (250 mg, 0.47 mmol), $(dba)_3Pd_2.CHCl_3$ (36 mg, 0.03 mmol), dichloromethane (2 mL), (*E*)-3-phenylprop-1-enylboronic acid (152 mg, 0.94 mmol), THF (6 mL) and triethylamine (0.2 mL, 1.43 mmol). Reaction time: 15 h. Eluent for chromatography: (35%) dicloromethane/hexane. Yield: 142 mg (60%). Orange oil. $[\alpha]_D^{20} = -196^o$ (c 1.2, in $CHCl_3$). $^1H$ NMR (250 MHz, $CDCl_3$) δ 7.15 (m, 5H, 5xArH), 5.86 (m, 3H, 3xH), 4.46 (dd, 1H, J= 5.4 and 3.0 Hz, H-5), 4.25 (d, 1H, J= 3.0 Hz, H-4), 3.35 (d, 2H, J= 6.2 Hz, $CH_2Ar$), 2.35 (d, 1H, J= 10.5 Hz, H-$6_{aX}$), 2.28 (m, 1H, H-$6_{eq}$, 0.86 (s, 9H, C($CH_3$)$_3$), 0.78 (s, 9H, C($CH_3$)$_3$), 0.12 (s, 3H, $CH_3$), 0.08 (s, 3H, $CH_3$), 0.05 (s, 3H, $CH_3$) and -0.03 (s, 3H, $CH_3$) ppm. $^1H$ NMR (63 MHz, $CDCl_3$) δ 175.5 (C), 139.3 (C), 136.1 (C), 132.1 (CH), 131.6 (CH), 128.7 (2xCH), 128.6 (CH), 128.5 (2xCH), 126.2 (CH), 75.8 (CH), 74.9 (C), 66.2 (CH), 39.2 ($CH_2$), 37.0 ($CH_2$), 25.6 (2xC(*C*$H_3$)$_3$), 18.0 (*C*($CH_3$)$_3$), 17.9 (*C*($CH_3$)$_3$), -3.1 ($2xSiCH_3$), -4.1 ($SiCH_3$) and -4.6 ($SiCH_3$) ppm. IR (film): 1800 (C=O) $cm^{-1}$. MS (Cl) *mlz* (%) 501 ($MH^+$). HRMS calcd for $C_{28}H_{45}O_4Si_2$ ($MH^+$): 501.2856; found: 501.2851.

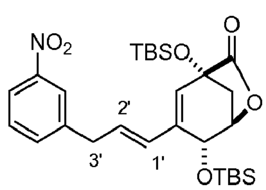

<u>*Example 4:*</u> *(1R,4R,5R)-1,4-di(butyldimethylsilyloxy)-3-[3-(3'-nitrophenyl)prop-1-enyl)cyclohex-2-en-1,5-carbolactone* ***(E).*** A solution of *(1R, 4S, 5R)*-1,4-di(tert-butyldimethylsilyloxy)cyclohex-2-en-1,5-carbolactone trifluoromethane sulphonate **A** (200 mg, 0.38 mmol), $Pd(PPh_3)_4$ (43 mg, 0.04 mmol), LiCl (48 mg, 1.13 mmol) and (*E*)-tributyl(3-(3-nitrophenyl)prop-1-enyl)stannane **F** (221 mg, 0.49 mmol) in dry THF (2 mL) was heated under reflux for 20 h. The

reaction mixture was cooled to room temperature and then diluted with diethyl ether and water. The organic layer was separated and the aqueous phase was extracted with diethyl ether (x3). All the combined organic extracts were dried (anh. $Na_2SO_4$), filtered and concentrated under reduced pressure. The obtained residue was purified by flash chromatography eluting with (65:35) dichloromethane/hexane to afford nitro derivative **E** (105 mg, 51%) as a colourless oil. $^1H$ NMR (400 MHz, $CDCl_3$) δ 8.08 (dt, 1H, *J* = 7.6 and 1.8 Hz, ArH), 8.04 (t, 1H, *J* = 1.8 Hz, ArH), 7.50 (dt, 1H, *J* = 8.0 and 1.8 Hz, ArH), 7.47 (t, 1H, *J* = 1.6 Hz, ArH), 5.96 (dt, 1H, *J* = 16.0 and 6.4 Hz, H-2'), 5.94 (br s, 1H, H-2), 5.87 (d, 1H, *J* = 16.0 Hz, H-1'), 4.53 (dd, 1H, *J* = 6.0 and 3.2 Hz, H-5), 4.30 (d, 1H, *J* = 3.2 Hz, H-4), 3.52 (d, 2H, *J* = 6.4 Hz, $ArCH_2$), 2.41 (d, 1H, J= 10.8 Hz, H-$6_{ax}$), 2.34 (ddd, 1H, J= 10.8, 6.0 and 2.0 Hz, H-6eq, 0.92 (s, 9H, $C(CH_3)_3$), 0.81 (s, 9H, $C(CH_3)_3$), 0.19 (s, 3H, $CH_3$), 0.14 (s, 3H, $CH_3$), 0.11 (s, 3H, $CH_3$) and 0.02 (s, 3H, $CH_3$) ppm. $^{13}C$ NMR (125 MHz, $CDCl_3$) δ 175.3 (C), 148.4 (C), 141.5 (C), 135.8 (C), 135.0 (CH), 133.2 (CH), 130.0 (CH), 129.6 (CH), 129.4 (CH), 123.6 (CH), 121.5 (CH), 75.7 (CH), 74.9 (C), 66.3 (CH), 38.8 ($CH_2$), 37.0 ($CH_2$), 25.6 (2x$C(CH_3)_3$), 18.0 ($C(CH_3)_3$), 17.5 ($C(CH_3)_3$), -3.1 (2x$SiCH_3$), -4.1 ($SiCH_3$) and -4.7 ($SiCH_3$) ppm. IR (film): 1793 (C=O) $cm^{-1}$. MS (ESI) *mlz* (%) 546 ($MH^+$). HRMS calcd for $C_{28}H_{44}O_6NSi_2$ ($MH^+$): 546.2702; found: 546.2698.

The (*E*)-tributyl(3-(3-nitrophenyl)prop-1-enyl)stannane required for the preparation of Example 4 was obtained from 1-bromomethyl-3-nitrobenzene as it is shown below:

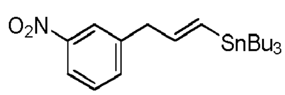

*Preparation of (E)-tributyl(3-(3-nitrophenyl)prop-1-enyl)stannane **(F).*** A solution of 1-bromomethyl-3-nitrobenzene (50 mg, 0.23 mmol) and $Pd(PPh_3)_4$ (8 mg, 7 μmol) in dry toluene (2 mL) was stirred for 15 min. (E)-1,2-bis (tributylstannyl)ethene (0.1 mL, 0.25 mmol) was then added and the resultant mixture was heated at 80 °C for 3 h. After cooling to room temperature diethyl ether and water was added. The organic layer was separated and the aqueous phase was extracted with diethyl ether (x3). All the combined organic extracts were dried (anh. $Na_2SO_4$), filtered and concentrated under reduced pressure. The crude residue was purified by flash chromatography eluting with (35%) dichloromethane/hexane to afford (*E*)-tributyl(3-(3-nitrophenyl)prop-1-enyl)stannane (28 mg, 27%) as a colourless oil. $^1H$ NMR (250 MHz, $CDCl_3$) δ 7.90 (m, 2H, 2xArH), 7.32 (m, 2H, 2xArH), 5.91 (m, 2H, CH=CH), 3.42 (m, 2H, $CH_2Ar$), 1.16 (m, 9H, 3x$CH_3$) and 0.73 (m, 18H, 9x$CH_2$) ppm.

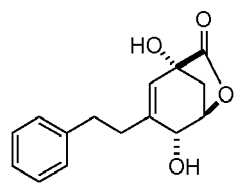

<u>Example 5</u>: *(1R, 4R, 5R)-1,4-dihydroxy-3-(2-phenylethyl)cyclohex-2-en-1, 5-carbolactone (**G**).* To a stirred solution of silylether **B** (71 mg, 0.14 mmol) in dry THF (2 mL), under argon at 0 °C, was added tetrabutylammonium fluoride (0.3 mL, 0.32 mmol, *ca* 1.0 M in THF). After 1 h, the ice-bath was removed and the reaction mixture was stirred for another hour. The solvent was evaporated and the obtained residue was dissolved in a mixture of ethyl acetate and water. The aqueous phase was acidified with dilute HCl and the organic layer was separated. The aqueous phase was extracted with ethyl acetate (x2). Al the combined organic extracts were dried (anh. $Na_2SO_4$), filtered and concentrated under reduced pressure. The obtained residue was purified by flash chromatography eluting with diethyl ether-hexanes [1°) 75:25, 2°) 100:0] to yield diol **G** (36 mg, 95%) as a pale yellow oil. $^1H$ NMR (300 MHz, $CD_3OD$) δ 7.23 (m, 2H, 2xArH), 7.13 (m, 3H, 3xArH), 5.67 (s, 1H, H-2), 4.58 (m, 1H, H-5), 4.01 (d, 1H, *J* = 2.9 Hz, H-4), 2.75 (m, 2H, $CH_2$), 2.43 (m, 2H, $CH_2$) and 2.25 (m, 2H, $CH_2$) ppm. $^{13}C$ NMR (75 MHz, $CD_3OD$) δ 179.1 (C), 142.6 (C), 140.7 (C), 131.3 (CH), 129.5 (2xCH), 129.4 (2xCH), 127.0 (CH), 78.0 (CH), 74.0 (C), 67.6 (CH), 37.5 ($CH_2$), 35.1 ($CH_2$) and 34.8 ($CH_2$) ppm. IR (film): 3514, 3405, 3366 and 3312 (O-H), 1783 (C=O) and 1757 (C=O) $cm^{-1}$.

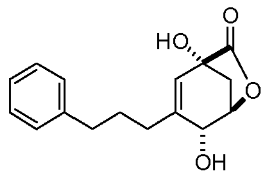

Example 6: *(1R,4R,5R)-1,4-dihydroxy-3-(3-phenylpropyl)cyclohex-2-en-1, 5-carbolactone (H).* The same experimental procedure as used for compound **B** (example 5), was applied to silyl ether **C** (105 mg, 0.21 mmol) in 3 mL of THF and 0.46 mL of tetrabutylammonium fluoride (0.46 mmol). Yield = 46 mg (80%). White solid. Mp: 77-79 °C. $[\alpha]_D^{20} = -197$ ° (c 1.1, in $CH_3OH$). $^1H$ NMR (250 MHz, $CD_3OD$) δ 7.18 (m, 2H, 2xArH), 7.09 (m, 3H, 3xArH), 5.69 (d, 1H, *J* = 1.1 Hz, H-2), 4.54 (m, 1H, H-5), 3.94 (d, 1H, J= 2.9 Hz, H-4), 2.51 (m, 2H, $CH_2$), 2.26 (m, 2H, $CH_2$), 2.08 (m, 2H, $CH_2$) and 1.69 (m, 2H, $CH_2$) ppm. $^{13}C$ NMR (63 MHz, $CD_3OD$) δ 179.3 (C), 143.2 (C), 141.2 (C), 130.8 (CH), 129.5 (2xCH), 129.4 (2xCH), 126.8 (CH), 78.0 (CH), 74.0 (C), 67.4 (CH), 37.5 ($CH_2$), 36.2 ($CH_2$), 32.5 ($CH_2$) and 29.9 ($CH_2$) ppm. IR (KBr): 3525, 3423, 3379 and 3316 (O-H), 1785 and 1754 (C=O) $cm^{-1}$. MS (CI) *m/z* (%) 275 ($MH^+$). HRMS calcd for $C_{16}H_{19}O_4$ ($MH^+$): 275.1283; found: 275.1285.

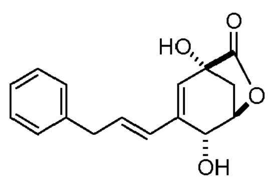

Example 7: *(1R,4R,5R)-1,4-dihydroxy-3-(3-phenylprop-1-enyl)cyclohex-2-en-1,5-carbolactone (**J**).* The same experimental procedure as used for compound **B** (example 5), was applied to silyl ether **D** (150 mg, 0.30 mmol) in 4 mL of THF and 0.66 mL of tetrabutylammonium fluoride (0.66 mmol). Yield = 78 mg (96%). White solid. Mp: 124-126 °C. $[\alpha]_D^{20} = -298^o$ (c 1.1, in $CH_3OH$). $^1H$ NMR (400 MHz, $CD_3OD$) δ 7.26 (m, 2H, 2xArH), 7.17 (m, 3H, 3xArH), 6.23 (dt, 1H, *J* = 15.6 and 6.8 Hz, H-2'), 6.00 (d, 1H, *J* = 15.6 Hz, H-1'), 5.89 (br s, 1H, H-2), 4.63 (m, 1H, H-5), 4.57 (br s, 1H, OH), 4.3 (d, 1H, *J* = 3.2 Hz, H-4), 3.43 (d, 2H, *J* = 6.8 Hz, $CH_2$), 2.3 δ (br d, 1H, *J* = 10.8 Hz, H-$6_{ax}$) and 2.34 (ddd, 1H, *J* = 10.8, 5.4 and 1.5 Hz, H-$6_{eq}$ ppm. $^{13}C$ NMR (100 MHz, $CD_3OD$) δ 178.6 (C), 141.2 (C), 138.0 (C), 133.4 (CH), 132.9 (CH), 130.2 (CH), 129.7 (2xCH), 129.5 (2xCH), 127.2 (CH), 78.0 (CH), 74.2 (C), 65.7 (CH), 40.2 ($CH_2$) and 37.6 ($CH_2$) ppm. IR (KBr): 3467 and 3321 (O-H), and 1753 (C=O) $cm^{-1}$. MS (CI) *mlz* (%) 273 ($MH^+$). HRMS calcd for $C_{16}H_{17}$O4 ($MH^+$): 273.1127; found: 273.1127.

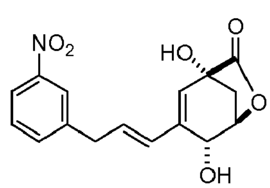

Example 8: *(1R,4R,5R)-1,4-dihydroxy-3-[3-(3'-nitrophenyl)prop-1-enyl]cyclohex-2-en-1,5-carbolactone (**K**).* The same experimental procedure as used for compound **B** (example 5), was applied to silyl ether **E** (103 mg, 0.19 mmol) in 3 mL of THF and 0.42 mL of tetrabutylammonium fluoride (0.42 mmol). Yield = 44 mg (73%). Yellow oil. $^1H$ NMR (250 MHz, $CD_3OD$) δ 8.00 (m, 2H, 2xArH), 7.55 (m, 1H, ArH), 7.47 (m, 1H, ArH), 6.16 (dt, 1H, J= 15.8 and 6.8 Hz, H-2'), 5.99 (d, 1H, J= 15.8 Hz, H-1'), 5.87 (s, 1H, H-2), 4.56 (m, 1H, H-5), 4.24 (d, 1H, *J* = 3.2 Hz, H-4), 3.51 (d, 2H, *J* = 6.8 Hz, $CH_2Ar$) and 2.32 (m, 2H, $CH_2$) ppm. $^{13}C$ NMR (63 MHz, $CD_3OD$) δ 178.6 (C), 149.8 (C), 143.7 (C), 137.8 (C), 136.2 (CH), 133.8 (CH), 131.8 (CH), 131.4 (CH), 130.7 (CH), 124.3 (CH), 122.3 (CH), 78.0 (CH), 74.3 (C), 65.7

(CH), 39.6 ($CH_2$) and 37.6 ($CH_2$) ppm. IR (film): 3434 (O-H) and 1784 (C=O) $cm^{-1}$. MS (ESI) *m/z* (%) 318 ($MH^+$). HRMS calcd for $C_{16}H_{16}O_6N$ ($MH^+$): 318.0972; found: 318.0981.

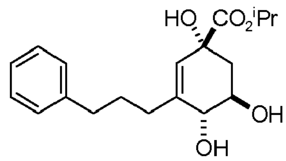

<u>*Example 9:*</u> *Isopropyl (1R, 4R, 5R)-1,4,5-trihydroxy-3(3-phenylpropyl)cyclohex-2-en-1-carboxylate **(1)***. A solution of the lactone **C** (38 mg, 0.14 mmol) in isopropanol (1.5 mL) was treated with $Ti(O^iPr)_4$ (50 μL, 0.17 mmol). The resultant mixture was heated at 80 °C for 4.5 hours. After cooling to room temperature, the reaction mixture was diluted with ethyl acetate and water. The organic layer was separated and the aqueous phase was extracted with ethyl acetate (x3). All the combined organic extracts were dried (anh. $Na_2SO_4$), filtered and concentrated under reduced pressure. The obtained residue was purified by flash chromatography eluting with (3:1) ethyl acetate/hexane to afford isopropyl ester **1** (16 mg, 34%) as colourless oil. $[\alpha]_D^{20} = -48^o$ (c0.2, in MeOH). $^1H$ NMR (250 MHz, $CD_3OD$) δ 7.21 (m, 5H, 5xArH), 5.43 (br s, 1H, H-2), 5.01 (m, 1H, C*H*$Me_2$), 3.88 (m, 2H, H-6), 2.72-2.38 (m, 4H, 2x$CH_2$), 2.11-1.97 (m, 2H, $CH_2$), 1.87-1.72 (m, 2H, $CH_2$) and 1.24 (d, 6H, *J* = 6.25 Hz, 2x$CH_3$) ppm. $^{13}C$ NMR (63 MHz, $CD_3OD$) δ 175.8 (C), 145.5 (C), 143.7 (C), 129.5 (2xCH), 129.3 (2xCH), 126.8 (CH), 124.4 (CH), 75.0 (C), 74.4 (CH), 70.9 (CH), 70.4 (CH), 40.8 ($CH_2$), 36.2 ($CH_2$), 32.9 ($CH_2$), 30.5 ($CH_2$), 22.0 ($CH_3$) and 21.9 ($CH_3$) ppm. IR (film) 3398 (O-H) and 1726 (C=O) $cm^{-1}$. MS (ESI) *m/z* (%) 357 ($MNa^+$). HRMS calculated for $C_{19}H_{26}O_5Na$ ($MNa^+$): 357.1672; found, 357.1662.

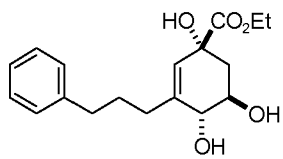

<u>*Example 10:*</u> *Ethyl (1R, 4R, 5R)-1,4,5-trihydroxy-3(3-phenylpropyl)cyclohex-2-en-1-carboxylate **(2).*** A solution of the lactone **C** (32.5 mg, 0.12 mmol) in dry ethanol (1.3 mL) was treated with sodium ethoxide (10.5 mg, 0.16 mmol). The resultant mixture was stirred at room temperature for 30 min and then it was diluted with ethyl acetate and water. The organic layer was separated and the aqueous phase was extracted with ethyl acetate (x3). All the combined organic extracts were dried (anh. $Na_2SO_4$), filtered and concentrated under reduced pressure. The obtained residue was purified by flash chromatography eluting with (3:1) ethyl acetate/hexane to afford ethyl ester **2** (17 mg, 45%) as colourless oil. $^1H$ NMR (250 MHz, $CD_3OD$) δ 7.20 (m, 5H, 5xArH), 5.45 (br s, 1H, H-2), 4.19 (q, 2H, *J* 7.3 Hz, $CH_2O$), 3.88 (t, 2H, H-4 + H-5), 2.61 (m, 2H, $CH_2$), 2.43 (m, 1H, C*H*H), 2.01 (m, 3H, CH*H*+CH2), 1.81 (m, 2H, $CH_2$) and 1.26 (t, 3H, *J* 7.3 Hz, $CH_3$) ppm. $^{13}C$ NMR (63 MHz, $CD_3OD$) δ 176.1 (C), 145.5 (C), 143.6 (C), 129.4 (2xCH), 129.2 (2xCH), 126.7 (CH), 124.2 (CH), 74.9 (CH), 74.4 (C), 70.8 (CH), 62.6 ($CH_2$), 40.7 ($CH_2$), 36.1 ($CH_2$), 32.9 ($CH_2$), 30.4 ($CH_2$) and 14.4 ($CH_3$) ppm. IR (film) 3398 (O-H) and 1733 (C=O) $cm^{-1}$. MS (ESI) *mlz* (%) 343 ($MNa^+$). HRMS calculated for $C_{18}H_{24}O_5Na$ ($MNa^+$): 343.1516; found, 343.1508.

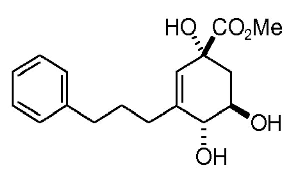

<u>*Example 11:*</u> *Methyl (1R, 4R, 5R)-1,4,5-trihydroxy-3(3-phenylpropyl)cyclohex-2-en-1-carboxylate **(3).*** A solution of the lactone C (50 mg, 0.18 mmol) in dry methanol (2 mL) was treated with potassium cyanide (13 mg, 0.20 mmol). The resultant mixture was stirred at room temperature for 1 hour and then it was diluted with ethyl acetate and water.

The organic layer was separated and the aqueous phase was extracted with ethyl acetate (x3). All the combined organic extracts were dried (anh. $Na_2SO_4$), filtered and concentrated under reduced pressure. The obtained residue was purified by flash chromatography eluting with (3:1) ethyl acetate/hexanes to afford methyl ester **3** (36 mg, 66%) and 13 mg of starting material (26%). White solid. $[\alpha]_D^{20} = -18^o$ (*c*1.3, in MeOH). $^1H$ NMR (250 MHz, $CD_3OD$) δ 7.21 (m, 5H, 5xArH), 5.44 (br s, 1H, H-2), 3.87 (m, 2H, H-4+H-5), 3.74 (s, 3H, OMe), 2.63 (m, 2H, $CH_2$), 2.39 (m, 1H, CH*H*), 2.05 (m, 3H, $CH_2$+C*H*H) and 1.87 (m, 2H, $CH_2$) ppm. $^{13}C$ NMR (63 MHz, $CD_3OD$) δ 176.6 (C), 145.8 (C), 143.6 (C), 129.5 (2xCH), 129.3 (2xCH), 126.8 (CH), 124.1 (CH), 75.0 (CH), 74.5 (C), 70.9 (CH), 53.1 ($CH_3$), 40.7 ($CH_2$), 36.3 ($CH_2$), 33.1 ($CH_2$) and 30.5 ($CH_2$) ppm. IR (KBr) 3388 (O-H) and 1736 (C=O) $cm^{-1}$. MS (ESI) *mlz* (%) 329 ($MNa^+$). HRMS calculated for $C_{17}H_{22}O_5Na$ ($MNa^+$): 329.1360; found, 329.1359.

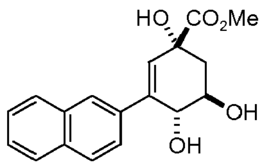

*Example 12: Methyl (1R, 4R, 5R)-1,4,5-trihydroxy-3-(naphthalen-2-yl)cyclohex-2-en-1-carboxylate (**21**).* A solution of the corresponding carbolactone of formula **V** (for the preparation of said carbolactone see González-Bello, C. ChemMedChem, 2007, 2, 194-207) (64 mg, 0.23 mmol) in dry methanol (2.6 mL) was treated with sodium methoxide (14 mg, 0.26 mmol). The resultant mixture was stirred at room temperature for 1 hour and then it was diluted with ethyl acetate and water. The organic layer was separated and the aqueous phase was extracted with ethyl acetate (x3). All the combined organic extracts were dried (anh. $Na_2SO_4$), filtered and concentrated under reduced pressure. The obtained residue was purified by flash chromatography eluting with (3:1) ethyl acetate/hexanes to afford methyl ester **21** (43 mg, 60%) and 22 mg of starting material (34%). White solid. $[\alpha]_D^{20} = -24^o$ (c1.1, in MeOH). $^1H$ NMR (250 MHz, $CD_3OD$) δ 7.86 (d, 1H, J= 1.3 Hz, ArH), 7.78 (m, 3H, 3xArH), 7.53 (dd, 1H, J= 8.5 and 1.8 Hz, ArH), 7.40 (m, 2H, 2xArH), 6.00 (t, 1H, *J* = 1.3 Hz, H-2), 4.57 (br s, 1H, O-H), 4.53 (dd, 1H, *J* = 1.5 and 7.0 Hz, H-4), 4.05 (m, 1H, H-5), 3.68 (s, 3H, $OCH_3$), 2.23 (dd, 1H, *J* = 13.3 and 10.8 Hz, H-$6_{ax}$) and 2.10 (ddd, 1H, *J* = 13.3, 3.8 and 1.3 Hz, H-$6_{eq}$) ppm. $^{13}C$ NMR (63 MHz, $CD_3OD$) δ 176.3 (C), 144.8 (C), 137.6 (C), 134.7 (C), 134.4 (C), 129.2 (CH), 128.5 (2xCH), 127.7 (CH), 127.2 (CH), 127.1 (CH), 127.0 (CH), 126.4 (CH), 74.0 (C), 73.4 (CH), 71.2 (CH), 53.1 ($OCH_3$) and 39.8 ($CH_2$) ppm. IR (KBr) 3444 (O-H), 3300 (O-H) and 1736 (C=O) $cm^{-1}$. MS (CI) *m*/*z* (%) 297 ($MH^+$-$H_2O$).

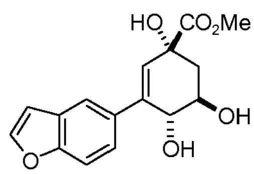

*Example 13: Methyl (1R, 4R, 5R)-3-(benzofuran-5-yl)-1,4,5-trihydroxycyclohex-2-en-1-carboxylate (22).* The experimental procedure used was the same as for compound **21.** The corresponding carbolactone of formula **V** used as starting material was obtained following the methods described in González-Bello, C. ChemMedChem, 2007, 2, 194-207. Yield: 33%. White solid. $[\alpha]_D^{20} = -19.8^o$ (*c*1.9, in MeOH). $^1H$ NMR (250 MHz, $CD_3OD$) δ 7.67 (d, 1H, *J* = 2.2 Hz, ArH), 7.62 (d, 1H, *J* = 1.7 Hz, ArH), 7.40 (br d, 1H, *J* = 8.6 Hz, ArH), 7.32 (dd, 1H, *J* = 1.7 and 8.6 Hz, ArH), 6.77 (d, 1H, *J* = 2.2 Hz, ArH), 5.86 (br s, 1H, H-2), 4.44 (dd, 1H, *J* = 7.1 and 1.4 Hz, H-4), 4.03 (m, 1H, H-5), 3.68 (s, 3H, $OCH_3$), 2.20 (dd, 1H, *J* = 10.6 and 13.2 Hz, H-$6_{ax}$) and 2.08 (ddd, 1H, *J* = 13.2, 3.9 and 1.1 Hz, H-$6_{eq}$) ppm. $^{13}C$ NMR (63 MHz, $CD_3OD$) δ 176.4 (C), 156.0 (C), 146.9 (CH), 145.2 (C), 135.2 (C), 128.7 (C), 126.9 (CH), 125.0 (CH), 121.0 (CH), 111.6 (CH), 107.7 (CH), 74.0 (C), 73.7 (CH), 71.2 (CH), 53.1 ($CH_3$) and 39.9 ($CH_2$) ppm. IR (KBr) 3429 (O-H) and 1718 (C=O) $cm^{-1}$. MS (ESI) *mlz* (%) 327 ($MNa^+$). HRMS calculated for $C_{16}H_{16}O_6Na$ ($MNa^+$): 327.0839; found, 327.0838.

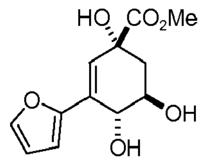

*Example 14: Methyl (1R, 4R, 5R)-3-(furan-2-yl)-1,4, 5-trihydroxycyclohex-2-en-1-carboxylate* ***(23).*** The experimental procedure used was the same as for compound **21.** The corresponding carbolactone of formula **V** used as starting material was obtained following the methods described in González-Bello, C. ChemMedChem, 2008, 3, 756-770. Yield: 29%. Beige solid. $[\alpha]_D^{20} = -5.65^o$ (*c*1.1, in MeOH). $^1H$ NMR (250 MHz, $CD_3OD$) δ 7.41 (d, 1H, *J* = 1.5 Hz, ArH), 6.62 (d, 1H, *J* = 3.3 Hz, ArH), 6.39 (dd, 1H, *J* =1.5 and 3.3 Hz, ArH), 6.20 (s, 1H, H-2), 4.17 (dd, 1H, *J* = 1.1 and 6.4 Hz, H-4), 3.97 (m, 1H, H-5), 3.69 (s, 3H, $OCH_3$), 2.17 (dd, 1H, *J* = 13.3 and 9.7 Hz, H-$6_{aX}$) and 2.03 (ddd, 1H, *J* = 13.3, 3.5 and 1.1 Hz, H-$6_{eq}$) ppm. $^{13}C$ NMR (63 MHz, $CD_3OD$) δ 176.2 (C), 153.2 (C), 143.3 (CH), 133.4 (C), 123.7 (CH), 112.3 (CH), 110.3 (CH), 73.6 (C), 72.0 (CH), 71.1 (CH), 53.1 ($CH_3$) and 39.1 ($CH_2$) ppm. IR (KBr) 3417 (O-H), 3342 (O-H) and 1736 (C=O) $cm^{-1}$. MS (ESI) *mlz* (%) 277 ($MNa^+$). HRMS calculated for $C_{12}H_{14}O_6Na$ ($MNa^+$): 277.0683; found, 277.0681.

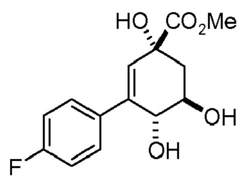

*Example 15: Methyl (1R, 4R, 5R)-3-(4-fluorophenyl)-1,4,5-trihydroxycyclohex-2-en-1-carboxylate* ***(24).*** The experimental procedure used was the same as for compound **21**. The corresponding carbolactone of formula **V** used as starting material was obtained following the methods described in González-Bello, C. J. Med. Chem. 2005, 48, 4871-4881. Yield: 29%. Colourless oil. $[\alpha]_D^{20} = -3.45$ (c.1.3, in MeOH). $^1H$ NMR (250 MHz, $CD_3OD$) δ 7.39-7.15 (m, 3H, 3xArH), 7.05-6.97 (m, 1H, ArH), 5.96 (s, 1H, H-2), 4.42 (dd, 1H, *J* = 1.5 and 7.0 Hz, H-4), 4.05 (m, 1H, H-5), 3.73 (s, 3H, $OCH_3$), 2.23 (dd, 1H, *J* = 10.5 and 13.5 Hz, H-$6_{ax}$) and 2.12 (ddd, 1H, *J* = 4.0, 1.0 and 13.5 Hz, H-$6_{eq}$) ppm. IR (KBr) 3421 (O-H) and 1734 (C=O) $cm^{-1}$. $^{13}C$ NMR (63 MHz, MeOD) δ 176.1 (C), 164.0 (C, *J* = 244 Hz), 143.7 (C), 142.9 (C), 130.8 (2xCH, *J* = 6 Hz), 128.2 (CH), 115.2 (2xCH, *J* = 25 Hz), 73.9 (C), 73.2 (CH), 71.0 (CH), 53.1 ($OCH_3$) and 39.7 ($CH_2$) ppm. MS (ESI) *mlz* (%) 305 ($MNa^+$). HRMS calculated for $C_{14}H_{15}O_5FNa$ ($MNa^+$): 305.0812; found, 305.0794.

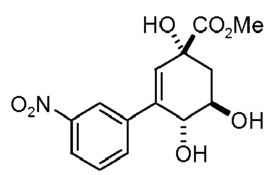

*Example 16: Methyl (1R, 4R, 5R)-1,4,5-trihydroxy-3-(3-nitrophenyl)cyclohex-2-en-1-carboxylate* ***(26).*** The experimental procedure used was the same as for compound **21**. The corresponding carbolactone of formula **V** used as starting material was obtained following the methods described in in González-Bello, C. J. Med. Chem. 2005, 48, 4871-4881. Yield: 23%. Colourless oil. $[\alpha]_D^{20} = -1.0^o$ (*c*1.0, in MeOH). $^1H$ NMR (250 MHz, $CD_3OD$) δ 8.27 (t, 1H, *J* = 2.0 Hz, ArH), 8.11 (ddd, 1H, *J* = 1.0, 2.0 and 8.0 Hz, ArH), 7.79 (ddd, 1H, *J* = 8.0, 2.0 and 1.0 Hz, ArH), 7.55 (t, 1H, *J* = 8.0 Hz, ArH), 6.03 (t, 1H, *J* = 1.3 Hz, H-2), 4.46 (dd, 1H, *J* = 7.3 and 1.5 Hz, H-4), 4.05 (m, 1H, H-5), 3.71 (s, 3H, $OCH_3$), 2.22 (dd, 1H, *J*= 10.5 and 13.3 Hz, H-$6_{ax}$) and 2.12 (ddd, 1H, *J* = 13.3, 1.3 and 4.3 Hz H-$6_{eq}$) ppm. $^{13}C$ NMR

(63 MHz, $CD_3OD$) δ 175.9 (C), 149.5 (C), 143.0 (C), 142.1 (C), 134.6 (CH), 130.3 (CH), 129.4 (CH), 123.3 (CH), 123.2 (CH), 73.9 (C), 73.2 (CH), 71.0 (CH), 53.2 ($CH_3$) and 39.8 ($CH_2$) ppm. IR (film) 3419 (O-H) and 1745 (C=O) $cm^{-1}$. MS (ESI) *m/z* (%) 332 ($MNa^+$). HRMS calculated for $C_{14}H_{15}O_7NaN$ ($MNa^+$): 332.0741; found, 332.0744.

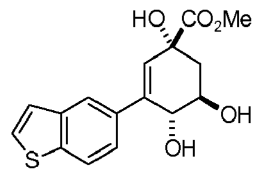

*<u>Example 17:</u> Methyl (1R, 4R, 5R)-3-(benzo[b]thiophen-5-yl)-1,4,5-trihydroxycyclohex-2-en-1-carboxylate (27).* The experimental procedure used was the same as for compound **21.** The corresponding carbolactone of formula **V** used as starting material was obtained following the methods described in González-Bello, C. ChemMedChem, 2007, 2, 194-207. Yield: 30%. White solid. $[\alpha]_D^{20} = -2.1^o$ (*c*1.0, in MeOH). $^1H$ NMR (250 MHz, $CD_3OD$) δ 7.85 (d, 1H, *J* = 1.8 Hz, ArH), 7.80 (d, 1H, *J* = 8.3 Hz, ArH), 7.49 (d, 1H, *J* = 5.5 Hz, ArH), 7.3 δ (dd, 1H, *J* = 1.8 and 8.3 Hz, ArH), 7.31 (br d, 1H, *J* = 5.5 Hz, ArH), 5.93 (t, 1H, *J* = 1.5 Hz, H-2), 4.47 (dd, 1H, *J* = 7.0 and 1.5 Hz, H-4), 4.05 (m, 1H, H-5), 3.68 (s, 3H, $OCH_3$), 2.21 (dd, 1H, *J* = 10.5 and 11.5 Hz, H-$6_{aX}$) and 2.09 (m, 1H, H-$6_{eq}$) ppm. $^{13}C$ NMR (63 MHz, $CD_3OD$) δ 176.3 (C), 145.0 (C), 141.1 (C), 140.4 (C), 136.6 (C), 127.9 (CH), 127.3 (CH), 125.1 (CH), 124.9 (CH), 123.3 (CH), 122.9 (CH), 74.0 (C), 73.6 (CH), 71.2 (CH), 53.1 ($OCH_3$) and 39.9 ($CH_2$) ppm. IR (KBr) 3435 (O-H) and 1720 (C=O) $cm^{-1}$. MS (ESI) *m/z* (%) 343 ($MNa^+$). HRMS calculated for $C_{16}H_{16}O_5NaS$ ($MNa^+$): 343.0611; found, 343.0616.

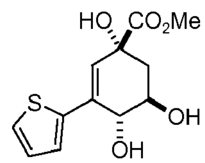

*<u>Example 18:</u> Methyl (1R, 4R, 5R)-1,4,5-trihydroxy-3-(thien-2-yl)cyclohex-2-en-1-carboxylate **(28).*** The experimental procedure used was the same as for compound **21.** The corresponding carbolactone of formula **V** used as starting material was obtained following the methods described in González-Bello, C. ChemMedChem, 2008, 3, 756-770. Yield: 7%. Colourless oil. $[\alpha]_D^{20} = -0.1^o$ (*c*0.8, in MeOH). $^1H$ NMR (250 MHz, $CD_3OD$) δ 7.24 (m, 2H, 2xArH), 6.95 (dd, 1H, *J* = 5.1 and 3.7 Hz, ArH), 6.07 (br s, 1H, H-2), 4.23 (dd, 1H, *J* = 1.1 and 6.2 Hz, H-4), 3.99 (m, 1H, H-5), 3.69 (s, 3H, $OCH_3$), 2.20 (dd, 1H, *J* = 13.3 and 9.5 Hz, H-$6_{ax}$) and 2.03 (ddd, 1H, *J* = 13.3, 3.4 and 1.1 Hz, H-$6_{eq}$) ppm. $^{13}C$ NMR (63 MHz, $CD_3OD$) δ 176.1 (C), 143.1 (C), 137.5 (C), 128.1 (CH), 126.7 (CH), 126.0 (CH), 125.9 (CH), 73.7 (C), 73.2 (CH), 71.2 (CH), 53.1 ($CH_3$) and 38.9 ($CH_2$) ppm. IR (film) 3365 (O-H) and 1732 (C=O) $cm^{-1}$.

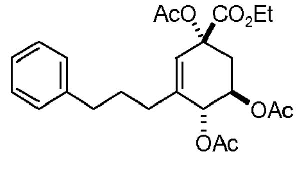

*<u>Example 19:</u> Ethyl (1R, 4R, 5R)-1,4,5-triacetoxy-3(3-phenylpropyl)cyclohex-2-en-1-carboxylate **(4).*** A solution of the triol **2** (10 mg, 31.25 μmol) in dry pyridine (0.5 mL) and acetic anhydride (0.5 mL) was stirred at room temperature for 24 h. The solvents were removed under reduced pressure and the obtained residue was purified by flash chromatography eluting with (2:3) diethyl ether/hexane to afford acetate **4** (10 mg, 77%) as colourless oil. $^1H$ NMR (250 MHz, $CDCl_3$) δ 7.30-7.25 (m, 2H, 2xArH), 7.21-7.13 (m, 3H, 3xArH), 6.03 (br s, 1H, H-2), 5.60 (br d, 1H, *J* = 8.3 Hz, H-4), 5.30

(m, 1H, H-5), 4.21 (q, 2H, *J* = 7.1 Hz, $OCH_2CH_3$), 2.60 (m, 2H, $CH_2$), 2.43 (dd, 1H, *J* = 14.0 and 4.8 Hz, H-$6_{eq}$), 2.18-2.04 (m, 3H, $CH_2$+H-$6_{ax}$), 2.08-2.00 (m, 2H, $CH_2$), 2.08 (s, 3H, AcO), 2.03 (s, 3H, AcO), 2.00 (s, 3H, AcO), 1.81-1.89 (m, 2H, $CH_2$) and 1.25 (t, 3H, *J* = 7.1 Hz, $OCH_2CH_3$) ppm. $^{13}C$ NMR (63 MHz, $CDCl_3$) δ 170.6 (C), 170.3 (C), 170.2 (C), 170.0 (C), 142.5 (C), 141.6 (C), 128.5 (2xC), 128.3 (2xCH), 125.9 (CH), 122.6 (CH), 77.4 (C), 72.0 (CH), 69.6 (CH), 62.0 ($OCH_2$), 35.2 ($CH_2$), 34.9 ($CH_2$), 31.5 ($CH_2$), 28.8 ($OCH_2$), 21.0 ($CH_3$), 20.9 ($CH_3$), 20.7 ($CH_3$) and 14.0 ($CH_3$) ppm. IR (NaCl) 1749 (C=O) and 1734 (C=O) $cm^{-1}$. MS (ESI) *m/z* (%) 469 ($MNa^+$). HRMS calculated for $C_{22}H_{31}O_8Na$ ($MNa^+$): 469.1833; found, 469.1839.

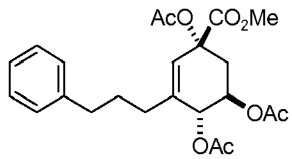

<u>Example 20:</u> *Methyl (1R, 4R, 5R)-1,4,5-triacetoxy-3(3-phenylpropyl)cyclohex-2-en-1-carboxylate (5).* The experimental procedure used was the same as for compound **4** (example 19), using compound **3** as starting material. Yield: 83%. Colourless oil. $^1H$ NMR (250 MHz, $CDCl_3$) δ 7.30-7.25 (m, 2H, 2xArH), 7.20-7.13 (m, 3H, 3xArH), 6.01 (br s, 1H, H-2), 5.58 (br d, 1H, *J* = 8.2 Hz, H-4), 5.30 (m, 1H, H-5), 3.75 (s, 3H, $OCH_3$), 2.60 (m, 2H, $CH_2$), 2.42 (dd, 1H, *J* = 13.8 and 4.2 Hz, H-$6_{eq}$), 2.19-2.00 (m, 3H, $CH_2$+H-$6_{aX}$), 2.08 (s, 3H, AcO), 2.03 (s, 3H, AcO), 2.00 (s, 3H, AcO) and 1.77 (m, 2H, $CH_2$) ppm. $^{13}C$ NMR (63 MHz, $CDCl_3$) δ 170.7 (C), 170.5 (C), 170.2 (C), 170.0 (C), 142.7 (C), 141.6 (C), 128.5 (2xCH), 128.3 (2xCH), 125.9 (CH), 122.4 (CH), 77.3 (C), 71.9 (CH), 69.5 (CH), 52.9 ($OCH_3$), 35.2 ($CH_2$), 34.8 ($CH_2$), 31.5 ($CH_2$), 28.8 ($CH_2$), 21.0 ($CH_3$), 20.9 ($CH_3$) and 20.7 ($CH_3$) ppm. IR (NaCl) 1751 (C=O) and 1736 (C=O) $cm^{-1}$. MS (ESI) *m/z* (%) 455 ($MNa^+$). HRMS calculated for $C_{23}H_{28}O_8Na$ ($MNa^+$): 455.1676; found, 455.1656.

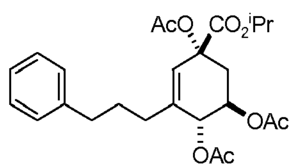

<u>Example 21:</u> *Isopropyl (1R, 4R, 5R)-1,4,5-triacetoxy-3(3-phenylpropyl)cyclohex-2-en-1-carboxylate **(6)**.* The experimental procedure used was the same as for compound **4** (example 19), using compound **1** as starting material. Yield: 73%. Colourless oil. $^1H$ NMR (250 MHz, $CDCl_3$) δ 7.43-7.25 (m, 2H, 2xArH), 7.21-7.14 (m, 3H, 3xArH), 6.04 (br s, 1H, H-2), 5.61 (dd, 1H, *J* = 8.3 and 1.7 Hz, H-4), 5.31 (m, 1H, H-5), 5.04 (quint, 1H, *J* 6.3 Hz, $CHMe_2$), 2.61 (m, 2H, $CH_2$), 2.42 (dd, 1H, *J* = 13.5 and 1.4 Hz, H-$6_{eq}$), 2.16-2.05 (m, 3H, $CH_2$+H-$6_{ax}$), 2.08 (s, 3H, AcO), 2.03 (s, 3H, AcO), 2.00 (s, 3H, AcO), 1.83-1.68 (m, 2H, $CH_2$) and 1.25 (d, 6H, *J* = 6.3 Hz, $OCH(CH_3)_2$) ppm. $^{13}c$ NMR (63 MHz, $CDCl_3$) δ 170.6 (C), 170.3 (C), 169.9 (C), 169.6 (C), 142.4 (C), 141.7 (C), 128.5 (2xCH), 128.3 (2xCH), 125.9 (CH), 122.8 (CH), 77.0 (C), 72.0 (CH), 69.7 (CH), 69.6 (CH), 35.1 ($CH_2$), 34.9 ($CH_2$), 31.4 ($CH_2$), 28.8 ($CH_2$), 21.5 ($OCH(CH_3)_2$), 21.0 ($CH_3$), 20.9 ($CH_3$) and 20.7 ($CH_3$) ppm. IR (NaCl) 1749 (C=O) and 1730 (C=O) $cm^{-1}$. MS (ESI) *m/z* (%) 483 ($MNa^+$). HRMS calculated for $C_{25}H_{32}O_8Na$ ($MNa^+$): 483.1989; found, 483.1985.

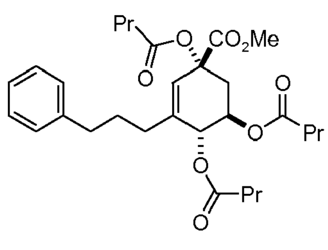

<u>Example 22:</u> *Methyl (1R, 4R, 5R)-1,4,5-tributyroxy-3(3-phenylpropyl)cyclohex-2-en-1-carboxylate* **(7)**. The experimental procedure used was the same as for compound **4** (example 19), using compound **3** as starting material and propionic anhydride instead of acetic anhydride. Yield: 49%. Colourless oil. $[\alpha]_D^{20} = +0.8^o$ (*c*1.0, in acetone). $^1H$

NMR (250 MHz, $CDCl_3$) δ 7.27 (m, 2H, 2xArH), 7.16 (m, 3H, 3xArH), 6.00 (s, 1H, H-2), 5.65 (br d, 1H, *J* = 8.4 Hz, H-4), 5.29 (m, 1H, H-5), 3.74 (s, 3H, OMe), 2.56-2.07 (m, 10H, $CH_2$), 1.70-1.56 (m, 10H) and 0.98-0.89 (m, 10H) ppm. $^{13}C$ NMR (63 MHz, $CDCl_3$) δ 173.0 (C), 172.8 (C), 172.7 (C), 170.8 (C), 142.9 (C), 141.6 (C), 128.5 (2xCH), 128.3 (2xCH), 125.8 (CH), 122.3 (CH), 77.2 (C), 71.5 (CH), 69.3 (CH), 52.8 ($OCH_3$), 36.1 ($CH_2$), 36.0 ($CH_2$), 35.9 ($CH_2$), 35.2 ($CH_2$), 34.9 ($CH_2$), 31.5 ($CH_2$), 28.8 ($CH_2$), 18.3 ($3xCH_2$), 13.6 ($CH_3$), 13.6 ($CH_3$) and 13.5 ($CH_3$) ppm. MS (ESI) *m/z* (%) 539 ($MNa^+$). HRMS calculated for $C_{29}H_{40}O_8Na$ ($MNa^+$): 539.2615; found, 539.2615.

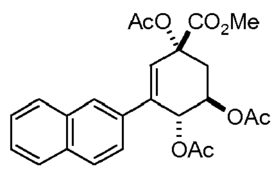

<u>Example 23:</u> *Methyl (1R, 4R, 5R)-1,4,5-triacetoxy-3-(naphthalen-2-yl)cyclohex-2-en-1-carboxylate **(8)**.* The experimental procedure used was the same as for compound **4** (example 19), using compound **21** as starting material. Yield: 69%. Colourless oil. $[\alpha]_D^{20}=$ -0.9° (c0.8, in acetone). $^1H$ NMR (250 MHz, $CDCl_3$) δ 7.79 (m, 5H, 5xArH), 7.48 (m, 2H, 2xArH), 7.39 (dd, 1H, *J* = 8.6 and 1.8 Hz, ArH), 6.60 (br s, 1H, H-2), 6.30 (dd, 1H, *J* = 7.7 and 1.7 Hz, H-4), 5.53 (m, 1H, H-5), 3.76 (s, 3H, $OCH_3$), 2.55 (dd, 1H, *J* = 13.5, 4.0 and 1.0 Hz, H-$6_{eq}$), 2.38 (dd, 1H, *J* = 13.5 and 11.8 Hz, H-$6_{ax}$), 2.20 (s, 3H, Ac), 2.08 (s, 3H, Ac) and 1.78 (s, 3H, Ac) ppm. $^{13}C$ NMR (63 MHz, $CDCl_3$) δ 170.5 (2xC), 170.2 (C), 170.1 (C), 142.7 (C), 134.2 (C), 133.1 (C), 133.1 (C), 128.2 (CH), 128.0 (CH), 127.6 (CH), 126.4 (2xCH), 125.9 (CH), 125.4 (CH), 124.2 (CH), 77.1 (C), 70.6 (CH), 69.7 (CH), 53.0 ($OCH_3$), 34.9 ($CH_2$), 21.0 ($2xCH_3$) and 20.6 ($CH_3$) ppm. IR (KBr): 1745 (C=O) $cm^{-1}$. MS (ESI) *m/z* (%) 463 ($MNa^+$). HRMS calculated for $C_{24}H_{24}O_8Na$ ($MNa^+$): 463.1363; found, 463.1364.

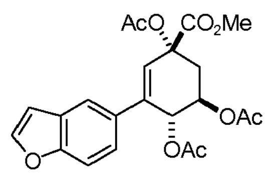

<u>Example 24:</u> *Methyl (1R, 4R, 5R)-1,4,5-triacetoxy-3-(benzofuran-5-yl)cyclohex-2-en-1-carboxylate **(9)**.* The experimental procedure used was the same as for compound **4** (example 19), using compound **22** as starting material. Yield: 73%. White solid. $[\alpha]_D^{20}=$ -1.6° (*c*1.1, in acetone). $^1H$ NMR (250 MHz, $CDCl_3$) δ 7.63 (d, 1H, *J* = 2.2 Hz, ArH), 7.53 (d, 1H, *J* = 1.8 Hz, ArH), 7.44 (d, 1H, *J* = 8.6 Hz, ArH), 7.20 (dd, 1H, *J* = 1.8 and 8.6 Hz, ArH), 6.75 (dd, 1H, *J* = 2.2 and 0.9 Hz, ArH), 6.46 (t, 1H, *J* = 1.4 Hz, H-2), 6.22 (dd, 1H, *J* = 7.8 and 1.8 Hz, H-4), 5.50 (m, 1H, H-5), 3.75 (s, 3H, $OCH_3$), 2.53 (ddd, 1H, *J* = 13.6, 4.2 and 1.4 Hz, H-$6_{eq}$), 2.35 (dd, 1H, *J* = 13.6 and 11.8 Hz, H-$6_{ax}$), 2.18 (s, 3H, $CH_3$), 2.07 (s, 3H, $CH_3$) and 1.79 (s, 3H, $CH_3$) ppm. $^{13}C$ NMR (63 MHz, $CDCl_3$) δ 170.6 (C), 170.5 (C), 170.2 (C), 170.1 (C), 154.9 (C), 145.7 (CH), 143.0 (C), 131.8 (C), 127.5 (C), 124.8 (CH), 123.0 (CH), 119.6 (CH), 111.2 (CH), 106.7 (CH), 77.2 (C), 70.8 (CH), 69.8 (CH), 53.0 ($OCH_3$), 34.9 ($CH_2$), 29.7 ($CH_2$), 21.0 ($2xCH_3$) and 20.7 ($CH_3$) ppm. IR (KBr) 1747 (C=O) $cm^{-1}$. MS (ESI) *m/z* (%) 453 ($MNa^+$). HRMS calculated for $C_{22}H_{22}O_9Na$ ($MNa^+$): 453.1156; found, 453.1158.

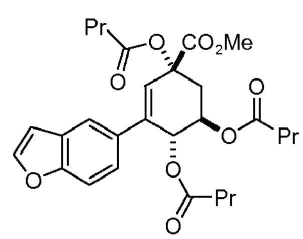

*<u>Example 25:</u> Methyl (1R, 4R, 5R)-3-(benzofuran-5-yl)-1,4,5-tributyroxycyclohex-2-en-1-carboxylate* ***(10)***. The experimental procedure used was the same as for compound **4** (example 19), using compound **22** as starting material and propionic anhydride instead of acetic anhydride. Yield: 87%. Colourless oil. $[\alpha]_D^{20} = -0.4^{o}$ (*c*1.1, in acetone). $^1H$ NMR (250 MHz, $CDCl_3$) δ 7.61 (d, 1H, *J* = 2.2 Hz, ArH), 7.51 (d, 1H, *J* = 1.8 Hz, ArH), 7.43 (d, 1H, *J* = 8.6 Hz, ArH), 7.19 (dd, 1H, *J* = 1.8 and 8.6 Hz, ArH), 6.74 (d, 1H, *J* = 2.2 Hz, ArH), 6.44 (br s, 1H, H-2), 6.27 (dd, 1H, *J* = 7.9 and 1.7 Hz, H-4), 5.49 (m, 1H, H-5), 3.74 (s, 3H, $OCH_3$), 2.55 (m, 1H, H-$6_{eq}$), 2.42 (t, 2H, *J* = 7.3 Hz, $CH_2$), 2.29 (m, 3H, H-$6_{ax}$+$CH_2$), 1.98 (m, 2H, $CH_2$), 1.80-1.56 (m, 4H, 2x$CH_2$), 1.30 (m, 2H, $CH_2$), 1.00 (t, 3H, *J* = 7.3 Hz, $CH_3$), 0.93 (t, 3H, *J* = 7.3 Hz, $CH_3$) and 0.65 (t, 3H, *J* = 7.3 Hz, $CH_3$) ppm. $^{13}C$ NMR (63 MHz, $CDCl_3$) δ 172.9 (C), 172.8 (2xC), 170.7 (C), 154.9 (C), 145.6 (CH), 143.3 (C), 131.9 (C), 127.5 (C), 124.8 (CH), 123.2 (CH), 119.6 (CH), 111.1 (CH), 106.7 (CH), 77.0 (C), 70.5 (CH), 69.7 (CH), 52.9 ($OCH_3$), 36.2 ($CH_2$), 36.0 ($CH_2$), 35.9 ($CH_2$), 35.0 ($CH_2$), 18.4 ($CH_2$), 18.3 ($CH_2$), 18.1 ($CH_2$), 13.6 ($CH_3$), 13.5 ($CH_3$) and 13.3 ($CH_3$) ppm. IR (film) 1745 (C=O) $cm^{-1}$. MS (ESI) *m*/*z* (%) 537 ($MNa^+$). HRMS calculated for $C_{28}H_{34}O_9Na$ ($MNa^+$): 537.2095; found, 537.2095.

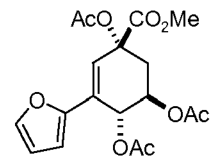

*<u>Example 26:</u> Methyl (1R, 4R, 5R)-1,4,5-triacetoxy-3-(furan-2-yl)cyclohex-2-en-1-carboxylate* ***(11)***. The experimental procedure used was the same as for compound **4** (example 19), using compound **23** as starting material. Yield: 84%. Beige solid. $[\alpha]_D^{20} =$ *+4.0˚ (c* 0.5,in acetone). $^1H$ NMR (250 MHz, $CDCl_3$) δ 7.40 (d, 1H, *J* = 1.6 Hz, ArH), 6.77 (br s, 1H, H-2), 6.39 (dd, 1H, *J* = 1.6 and 3.4 Hz, ArH), 6.33 (d, 1H, *J* = 3.4 Hz, ArH), 5.90 (dd, 1H, *J* = 0.8 and 5.9 Hz, H-4), 5.37 (m, 1H, H-5), 3.76 (s, 3H, $OCH_3$), 2.42 (m, 2H, H-6), 2.12 (s, 3H, AcO), 2.05 (s, 3H, AcO) and 2.04 (s, 3H, AcO) ppm. $^{13}C$ NMR (63 MHz, $CDCl_3$) δ 170.6 (C), 170.3 (C), 170.0 (2xC), 150.3 (C), 143.0 (CH), 130.3 (C), 121.8 (CH), 111.5 (CH), 108.6 (CH), 76.7 (C), 69.3 (CH), 67.9 (CH), 53.0 ($CH_3$), 33.7 ($CH_2$), 20.9 (3x$CH_3$) ppm. IR (film) 1734 (C=O) $cm^{-1}$. MS (ESI) *m*/*z* (%) 403 ($MNa^+$). HRMS calculated for $C_{18}H_{20}O_9Na$ ($MNa^+$): 403.1000; found, 403.0998.

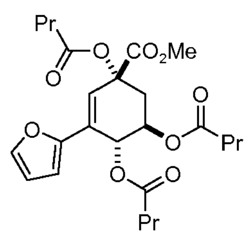

*<u>Example 27:</u> Methyl (1R, 4R, 5R)-1,4,5-triburyroxy-3-(furan-2-yl)cyclohex-2-en-1-carboxylate* ***(12).*** The experimental procedure used was the same as for compound **4** (example 19), using compound **23** as starting material and propionic anhydride instead of acetic anhydride. Yield: 88%. Colourless oil. $^1H$ NMR (250 MHz, $CDCl_3$) δ 7.38 (d, 1H, *J* = 0.8 Hz, ArH), 6.76 (s, 1H, H-2), 6.37 (dd, 1H, *J* = 1.8 and 3.4 Hz, ArH), 6.31 (d, 1H, J = 3.4 Hz, ArH), 5.96 (d, 1H, *J* = 6.4 Hz, H-4), 5.39 (m, 1H, H-5), 3.75 (s, 3H, $OCH_3$), 2.42-2.22 (m, 8H, 4x$CH_2$), 1.64 (m, 6H, 3x$CH_2$) and 0.93 (m, 9H, 3x$CH_3$) ppm. $^{13}C$ NMR (63 MHz, $CDCl_3$) δ 172.9 (C), 172.7 (2xC), 170.7 (C), 150.3 (C), 142.9 (CH), 130.7 (C), 121.9 (CH), 111.4 (CH), 108.6 (CH), 76.5 (C), 69.4 (CH), 68.0 (CH), 52.9 ($CH_3$), 36.1 ($CH_2$), 36.0 ($CH_2$), 35.9 ($CH_2$), 34.1 ($CH_2$), 18.3 ($CH_2$), 18.2 ($CH_2$), 18.2 ($CH_2$), 13.6 (2x$CH_3$) and 13.5 ($CH_3$) ppm. IR (film) 1743 (C=O) $cm^{-1}$. MS (ESI) *m*/*z* (%) 487 ($MNa^+$). HRMS calculated for $C_{24}H_{32}O_9Na$ ($MNa^+$): 487.1939; found, 487.1938.

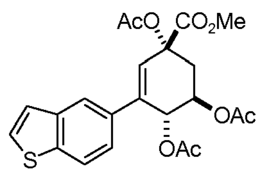

_Example 28:_ *Methyl (1R, 4R, 5R)-1,4,S-triacetoxy-3-(benzo[b]thiophen-5-yl)cyclohex-2-en-1-carboxylate* ***(13).*** The experimental procedure used was the same as for compound **4** (example 19), using compound **27** as starting material. Yield: 90%. Colourless oil. $[\alpha]_D^{20} = -0.6^o$ (c0.8, in $CHCl_3$). $^1H$ NMR (250 MHz, $CDCl_3$) δ 7.81 (d, 1H, *J* = 8.3 Hz, ArH), 7.75 (br s, 1H, ArH), 7.45 (d, 1H, *J* = 5.3 Hz, ArH), 7.31 (d, 1H, *J* = 5.3 Hz, ArH), 7.24 (m, 1H, ArH), 6.52 (s, 1H, H-2), 6.25 (dd, 1H, *J* = 7.7 and 1.6 Hz, H-4), 5.51 (m, 1H, H-5), 3.75 (s, 3H, $OCH_3$), 2.53 (dd, 1H, *J* = 13.5 and 4.1 Hz, H-$6_{eq}$), 2.36 (dd, 1H, *J* = 13.5 and 11.9 Hz, H-$6_{ax}$), 2.18 (s, 3H, $CH_3$), 2.07 (s, 3H, $CH_3$) and 1.79 (s, 3H, $CH_3$) ppm. $^{13}C$ NMR (63 MHz, $CDCl_3$) δ 170.5 (2xC), 170.2 (C), 170.1 (C), 142.7 (C), 139.8 (CH), 139.6 (C), 133.1 (C), 127.2 (CH), 125.1 (CH), 123.9 (CH), 122.6 (CH), 122.3 (CH), 121.7 (CH), 77.1 (C), 70.7 (CH), 69.7 (CH), 53.0 ($OCH_3$), 34.8 ($CH_2$), 20.9 (2x$CH_3$) and 20.6 ($CH_3$) ppm. IR (KBr) 1751 (C=O) and 1734 (C=O) $cm^{-1}$. MS (ESI) *m/z* (%) 553 ($MNa^+$). HRMS calculated for $C_{28}H_{34}O_8SNa$ ($MNa^+$): 553.1867; found, 553.1868.

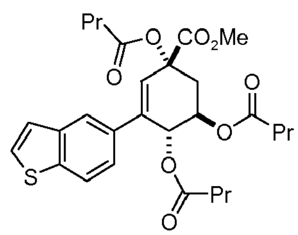

_Example 29:_ *Methyl (1R, 4R, 5R)-3-(benzo[b]thiophen-5-yl)-1,4,5-tributyroxycyclohex-2-en-1-carboxylate* ***(14).*** The experimental procedure used was the same as for compound **4** (example 19), using compound **27** as starting material and propionic anhydride instead of acetic anhydride. Yield: 70%. Colourless oil. $[\alpha]_D^{20} = -1.7^o$ (c2.5, in $CHCl_3$). $^1H$ NMR (250 MHz, $CDCl_3$) δ 7.80 (d, 1H, *J* = 8.4 Hz, ArH), 7.72 (br s, 1H, ArH), 7.44 (d, 1H, *J* = 5.3 Hz, ArH), 7.30 (d, 1H, J = 5.3 Hz, ArH), 7.24 (m, 1H, ArH), 6.50 (s, 1H, H-2), 6.30 (dd, 1H, *J* = 8.0 and 1.1 Hz, H-4), 5.50 (m, 1H, H-5), 3.74 (s, 3H, $OCH_3$), 2.55 (dd, 1H, *J* = 13.8 and 3.9 Hz, H-$6_{eq}$), 2.42 (t, 2H, *J* = 7.3 Hz, $CH_2$), 2.29 (m, 3H, H-$6_{ax}$+$CH_2$), 1.98 (m, 2H, $CH_2$), 1.80-1.56 (m, 4H, 2x$CH_2$), 1.31 (m, 2H, $CH_2$), 1.00 (t, 3H, *J* = 7.3 Hz, $CH_3$), 0.93 (t, 3H, *J* = 7.3 Hz, $CH_3$) and 0.64 (t, 3H, *J* = 7.3 Hz, $CH_3$) ppm. $^{13}C$ NMR (63 MHz, $CDCl_3$) δ 172.9 (C), 172.8 (2xC), 170.6 (C), 143.1 (C), 139.7 (C), 139.6 (C), 133.2 (C), 127.1 (CH), 125.1 (CH), 123.9 (CH), 122.8 (CH), 122.3 (CH), 121.8 (CH), 77.0 (C), 70.4 (CH), 69.6 (CH), 52.9 ($OCH_3$), 36.1 ($CH_2$), 35.9 ($CH_2$), 35.9 ($CH_2$), 35.0 ($CH_2$), 18.4 ($CH_2$), 18.2 ($CH_2$), 18.0 ($CH_2$), 13.6 ($CH_3$), 13.5 ($CH_3$) and 13.3 ($CH_3$) ppm. IR (film) 1741 (C=O) $cm^{-1}$. MS (ESI) *m/z* (%) 469 ($MNa^+$). HRMS calculated for $C_{22}H_{22}O_8SNa$ ($MNa^+$): 469.0928; found, 469.0927.

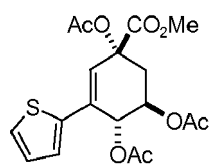

_Example 30:_ *Methyl (1R,4R,5R)-1,4,5-triacetoxy-3-(thien-2-yl)cyclohex-2-en-1-carboxylate* ***(15).*** The experimental procedure used was the same as for compound **4** (example 19), using compound **28** as starting material. Yield:

94%. Colourless oil. $[\alpha]_D^{20} = +0.8^o$ (*c*1.0, in $CHCl_3$). $^1H$ NMR (250 MHz, $CDCl_3$) δ 7.26 (dd, 1H, *J* = 5.0 and 1.0 Hz, ArH), 7.03 (dd, 1H, *J* = 3.8 and 1.0 Hz, ArH), 6.98 (dd, 1H, *J* = 5.0 and 3.8 Hz, ArH), 6.60 (br s, 1H, H-2), 5.97 (dd, 1H, *J* = 6.5 and 1.3 Hz, H-4), 5.42 (m, 1H, H-5), 3.76 (s, 3H, $OCH_3$), 2.41 (m, 2H, H-6), 2.13 (s, 3H, $CH_3$), 2.05 (s, 3H, $CH_3$) and 2.02 (s, 3H, $CH_3$) ppm. $^{13}C$ NMR (63 MHz, $CDCl_3$) δ 170.5 (C), 170.4 (C), 170.1 (2xC), 139.8 (C), 135.0 (C), 127.5 (CH), 125.9 (CH), 125.5 (CH), 123.9 (CH), 76.8 (C), 69.8 (CH), 69.4 (CH), 53.0 ($OCH_3$), 34.0 ($CH_2$) and 21.0 ($2xCH_3$) ppm. MS (ESI) *m*/*z* (%) 419 ($MNa^+$). HRMS calculated for $C_{18}H_{20}O_8SNa$ ($MNa^+$): 419.0771; found, 419.0764.

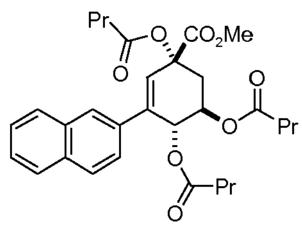

*<u>Example 31:</u> Methyl (1R, 4R, 5R)-1,4,5-tributyroxy-3-(naphthalen-2-yl)cyclohex-2-en-1-carboxylate* ***(16).*** The experimental procedure used was the same as for compound **4** (example 19), using compound **21** as starting material and propionic anhydride instead of acetic anhydride. Yield: 80%. Colourless oil. $[\alpha]_D^{20} = -0.7^o$ (*c*1.0, in acetone). $^1H$ NMR (250 MHz, $CDCl_3$) δ 7.79 (m, 4H, 4xArH), 7.47 (m, 2H, 2xArH), 7.38 (dd, 1H, J = 8.6 and 1.5 Hz, ArH), 6.57 (s, 1H, H-2), 6.35 (dd, 1H, *J* = 8.0 and 1.6 Hz, H-4), 5.52 (m, 1H, H-5), 3.75 (s, 3H, OMe), 2.57 (dd, 1H, *J* = 13.8 and 3.8 Hz, $H-6_{eq}$), 2.44 (t, 2H, *J* = 7.4 Hz, $CH_2$), 2.30 (t, 2H, *J* = 7.4 Hz, $CH_2$), 2.08-1.92 (m, 3H), 1.79-1.57 (m, 4H), 1.35-1.25 (m, 3H), 1.02 (t, 3H, *J* = 7.4 Hz, $CH_3$), 0.94 (t, 3H, *J* = 7.4 Hz, $CH_3$) and 0.63 (t, 3H, *J* = 7.4 Hz, $CH_3$) ppm. $^{13}C$ NMR (63 MHz, $CDCl_3$) δ 173.0 (C), 172.8 (2xC), 170.6 (C), 143.0 (C), 134.3 (C), 133.1 (2xC), 128.2 (CH), 128.0 (CH), 127.5 (CH), 126.4 (2xCH), 125.9 (CH), 125.4 (CH), 124.4 (CH), 77.0 (C), 70.3 (CH), 69.7 (CH), 52.9 ($OCH_3$), 36.2 ($CH_2$), 36.0 ($CH_2$), 35.9 ($CH_2$), 35.0 ($CH_2$), 18.4 ($CH_2$), 18.3 ($CH_2$), 18.0 ($CH_2$), 13.6 ($CH_3$), 13.5 ($CH_3$) and 13.3 ($CH_3$) ppm. IR (film): 1738 (C=O) $cm^{-1}$. MS (ESI) *m*/*z* (%) 537 ($MNa^+$). HRMS calculated for $C_{30}H_{36}O_8Na$ ($MNa^+$): 547.2306; found, 547.2306.

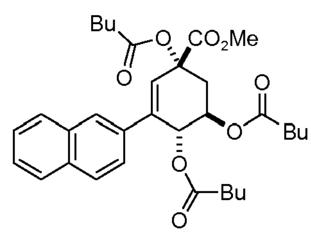

*<u>Example 32:</u> Methyl (1R, 4R, 5R)-3-(naphthalen-2-yl)-1,4,5-tripentyroxycyclohex-2-en-1-carboxylate* (***17***). The experimental procedure used was the same as for compound **4** (example 19), using compound **21** as starting material and butanoic anhydride instead of acetic anhydride. Yield: 92%. Colourless oil. $[\alpha]_D^{20} = -1.1^o$ (*c*1.9, in acetone). $^1H$ NMR (250 MHz, $CDCl_3$) δ 7.78 (m, 4H, 4xArH), 7.47 (dd, 2H, *J* = 6.3 and 3.3 Hz, 2xArH), 7.38 (dd, 1H, *J* = 8.5 and 1.5 Hz, ArH), 6.56 (br s, 1H, H-2), 6.34 (dd, 1H, *J* = 7.8 and 1.5 Hz, H-4), 5.52 (m, 1H, H-5), 3.75 (s, 3H, OMe), 2.59-2.40 (m, 4H, $2xCH_2$), 2.32 (t, 2H, *J* = 7.5 Hz, $CH_2$), 1.98 (m, 2H, $CH_2$), 1.76-1.53 (m, 4H, $2xCH_2$), 1.47-1.15 (m, 8H, $4xCH_2$), 0.94 (m, 6H, $2xCH_3$) and 0.55 (t, 3H, *J* = 7.4 Hz, $CH_3$) ppm. $^{13}C$ NMR (63 MHz, $CDCl_3$) δ 173.1 (C), 172.9 (2xC), 170.6 (C), 143.1 (C), 134.3 (C), 133.1 (C), 133.0 (C), 128.2 (CH), 128.0 (CH), 127.5 (CH), 126.3 (2xCH), 125.9 (CH), 125.4 (CH), 124.4 (CH), 77.0 (C), 70.4 (CH), 69.6 (CH), 52.9 ($OCH_3$), 35.0 ($CH_2$), 34.0 ($CH_2$), 33.8 ($2xCH_2$), 26.8 ($2xCH_2$), 26.7 ($CH_2$), 22.2 ($CH_2$), 22.1 ($CH_2$), 21.8 ($CH_2$), 13.7 ($2xCH_3$) and 13.3 ($CH_3$) ppm. IR (film): 1743 (C=O) $cm^{-1}$. MS (ESI) *m*/*z* (%) 589 ($MNa^+$). HRMS calculated for $C_{33}H_{42}O_8Na$ ($MNa^+$): 589.2772; found, 589.2772.

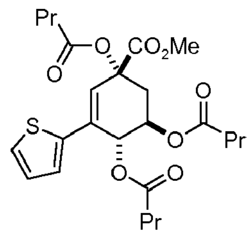

*Example 33: Methyl (1R, 4R, 5R)-1,4,5-tributyroxy-3-(thien-2-yl)cyclohex-2-en-1-carboxylate* ***(18).*** The experimental procedure used was the same as for compound **4** (example 19), using compound **28** as starting material and propionic anhydride instead of acetic anhydride. Yield: 92%. White solid. $[\alpha]_D^{20} = +0.2^o$ (*c*1.0, in $CHCl_3$). $^1H$ NMR (250 MHz, $CDCl_3$) δ 7.24 (dd, 1H, *J* = 5.0 and 1.3 Hz, ArH), 7.01 (dd, 1H, *J* = 3.5 and 1.3 Hz, ArH), 6.96 (dd, 1H, *J* = 5.0 and 3.5 Hz, ArH), 6.59 (br s, 1H, H-2), 6.03 (dd, 1H, *J* = 7.0 and 1.3 Hz, H-4), 5.43 (m, 1H, H-5), 3.76 (s, 3H, OMe), 2.48-2.18 (m, 8H, $4xCH_2$), 1.73-1.49 (m, 6H, $3xCH_2$) and 1.00-0.82 (m, 9H, $3xCH_3$) ppm. $^{13}C$ NMR (63 MHz, $CDCl_3$) δ 172.9 (C), 172.7 (2xC), 170.6 (C), 139.7 (C), 135.5 (C), 127.4 (CH), 125.8 (CH), 125.5 (CH), 124.2 (CH), 76.7 (C), 69.8 (CH), 69.5 (CH), 52.9 ($OCH_3$), 36.1 ($CH_2$), 36.1 ($CH_2$), 35.9 ($CH_2$), 34.4 ($CH_2$), 18.3 ($CH_2$), 18.2 ($2xCH_2$), 13.6 ($CH_3$), 13.5 ($CH_3$) and 13.5 ($CH_3$) ppm. MS (ESI) *m/z* (%) 503 ($MNa^+$). HRMS calculated for $C_{24}H_{32}O_8NaS$ ($MNa^+$): 503.1710; found, 503.1709.

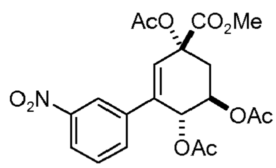

*Example 34: Methyl (1R, 4R, 5R)-1,4,5-triacetoxy-3-(3-nitrophenyl)cyclohex-2-en-1-carboxylate* ***(19).*** The experimental procedure used was the same as for compound **4** (example 19), using compound **26** as starting material. Yield: 65%. Colourless oil. $[\alpha]_D^{20} = +1.2^o$ (*c*1.0, in $CHCl_3$). $^1H$ NMR (250 MHz, $CDCl_3$) δ 8.16 (m, 2H, 2xArH), 7.54 (m, 2H, 2xArH), 6.55 (br s, 1H, H-2), 6.13 (dd, 1H, *J* = 7.5 and 1.5 Hz, H-4), 5.47 (m, 1H, H-5), 3.75 (s, 3H, $OCH_3$), 2.52 (dd, 1H, *J* = 3.3 and 13.5 Hz, H-$6_{eq}$), 2.31 (dd, 1H, J= 13.5 and 11.8 Hz, H-$6_{ax}$), 2.17 (s, 3H, $CH_3$), 2.05 (s, 3H, $CH_3$) and 1.83 (s, 3H, $CH_3$) ppm. $^{13}C$ NMR (63 MHz, $CDCl_3$) 8 170.2 (2xC), 170.1 (C), 170.0 (C), 148.2 (C), 140.5 (C), 138.6 (C), 132.6 (CH), 129.4 (CH), 127.5 (CH), 123.3 (CH), 121.9 (CH), 76.6 (C), 70.4 (CH), 69.3 (CH), 53.1 ($CH_3$), 34.8 ($CH_2$), 20.9 ($CH_3$), 20.8 ($CH_3$) and 20.5 ($CH_3$) ppm. MS (ESI) *mlz* (%) 458 ($MNa^+$). HRMS calculated for $C_{20}H_{21}O_{10}NNa$ ($MNa^+$): 458.1058; found, 458.1060.

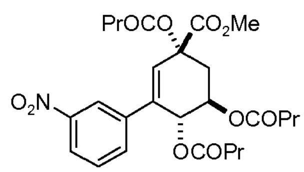

*Example 35: Methyl (1R, 4R, 5R)-1,4,5-tributyroxy-3-(3-nitrophenyl)cyclohex-2-en-1-carboxylate* ***(20)***. The experimental procedure used was the same as for compound **4** (example 19), using compound **26** as starting material and propionic anhydride instead of acetic anhydride. Yield: 90%. Colourless oil. $[\alpha]_D^{20} = +0.2^o$ (*c*1.0, in $CHCl_3$). $^1H$ NMR (250 MHz, $CDCl_3$) δ 8.15 (m, 2H, 2xArH), 7.53 (m, 2H, 2xArH), 6.53 (br s, 1H, H-2), 6.18 (dd, 1H, *J* = 8.0 and 1.8 Hz, H-4), 5.46 (m, 1H, H-5), 3.74 (s, 3H, $OCH_3$), 2.54 (dd, 1H, *J* = 4.0 and 13.5 Hz, H-$6_{eq}$), 2.44-2.34 (m, 5H, $2xCH_2$+H-$6_{ax}$), 2.02 (m, 2H, $CH_2$), 1.75-1.57 (m, 4H, $2xCH_2$), 1.35 (m, 2H, $CH_2$), 0.99 (t, 3H, J = 7.3 Hz, $CH_3$), 0.92 (t, 3H, *J* = 7.3

Hz, $CH_3$) and 0.68 (t, 3H, *J* = 7.3 Hz, $CH_3$) ppm. $^{13}C$ NMR (63 MHz, $CDCl_3$) δ 172.7 (3xC), 170.2 (C), 148.2 (C), 141.0 (C), 138.7 (C), 132.7 (CH), 129.3 (CH), 127.4 (CH), 123.2 (CH), 122.0 (CH), 76.5 (C), 70.1 (CH), 69.2 (CH), 53.0 ($CH_3$), 36.0 ($CH_2$), 35.8 ($CH_2$), 35.8 ($CH_2$), 34.9 ($CH_2$), 18.3 ($CH_2$), 18.2 ($CH_2$), 18.0 ($CH_2$), 13.6 ($CH_3$), 13.4 ($CH_3$) and 13.3 ($CH_3$) ppm. MS (ESI) *m/z* (%) 542 ($MNa^+$). HRMS calculated for $C_{26}H_{33}O_{10}NNa$ ($MNa^+$): 542.1997; found, 542.1997.

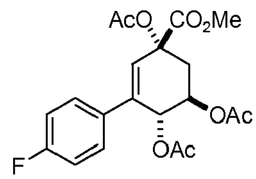

*<u>Example 36:</u> Methyl (1R, 4R, 5R)-1,4,5-triacetoxy-3-(4-fluorophenyl)-cyclohex-2-en-1-carboxylate* ***(25).*** The experimental procedure used was the same as for compound **4** (example 19), using compound **24** as starting material. Yield: 80%. Colourless oil. $^1H$ NMR (250 MHz, $CDCl_3$) δ 7.28 (m, 1H, ArH), 7.01 (m, 3H, 3xArH), 6.47 (s, 1H, H-2), 6.11 (dd, 1H, *J* = 1.5 and 7.5 Hz, H-4), 5.45 (m, 1H, H-5), 3.75 (s, 3H, $OCH_3$), 2.50 (d,1H, *J* = 2.5 and 13.5 Hz, H-$6_{ax}$), 2.31 (dd, 1H, *J* = 11.8 and 13.5 Hz, H-$6_{eq}$), 2.16 (s, 3H, AcO), 2.05 (s, 3H, AcO) and 1.84 (s, 3H, AcO) ppm. MS (ESI) *mlz* (%) 431 ($MNa^+$). HRMS calculated for $C_{20}H_{21}O_8FNa$ ($MNa^+$): 431.1118; found, 431.1118.

*<u>Example 37:</u> Effect of compounds of formula* ***I*** *of the invention on the bacterial growth of Mycobacterium tuberculosis*. The minimum inhibitory concentration (MIC) was measured against *Mycobacterium smegmatis mc²* and *Mycobacterium Bovis* BCG following the Alamar Blue Assay protocol described by Palomino, J. C. et al. Antimicrob. Agents Chemother. 2002, 46, 2720-2722. The results are summarized in Table 1.

All the compounds tested displayed inhibitory activity.

**Table 1.** MICs (μg/mL) for *Mycobacterium smegmatis mc²* and *Mycobacterium Bovis* BCG in the presence of compounds **I.** [a]

| **Compound** | | *M. smegmatis mc²* | *M. bovis* BCG |
|---|---|---|---|
| 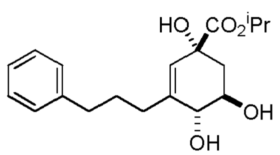 | **1** | >160 | >160 |
| 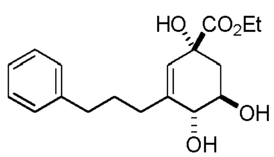 | **2** | >160 | >160 |
| 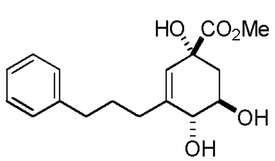 | **3** | >160 | >160 |
| 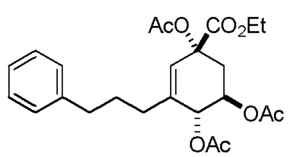 | **4** | >160 | 80 |

(continued)

| Compound | | *M. smegmatis mc*² | *M. bovis* BCG |
|---|---|---|---|
| 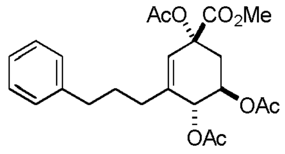 | **5** | >160 | 160 |
| 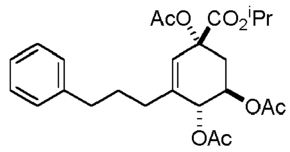 | **6** | >160 | >160 |
| 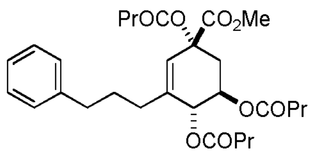 | **7** | >160 | >160 |
| 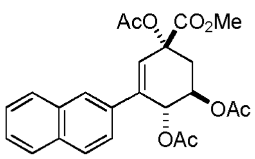 | **8** | >160 | 80 |
| 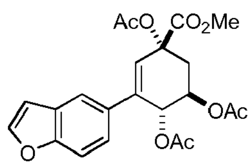 | **9** | >160 | 160 |
| 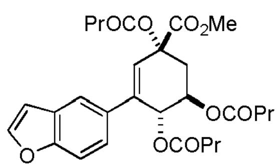 | **10** | >160 | >160 |
| 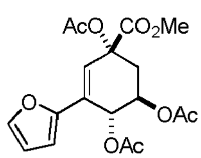 | **11** | >160 | 160 |
| 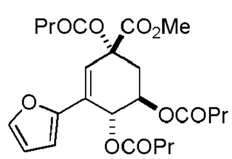 | **12** | >160 | >160 |

(continued)

| Compound | | *M. smegmatis mc*[2] | *M. bovis* BCG |
|---|---|---|---|
| 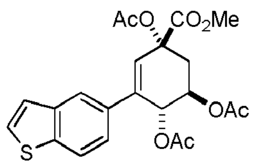 | **13** | >160 | >160 |
| 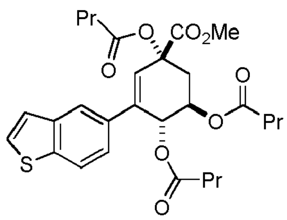 | **14** | >160 | >160 |
| 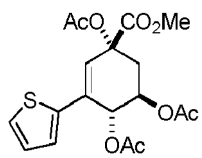 | **15** | >160 | >160 |
| 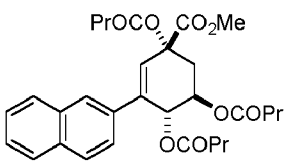 | **16** | >160 | >160 |
| 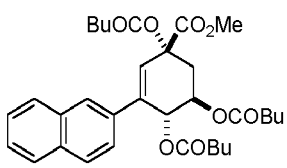 | **17** | >160 | >160 |
| 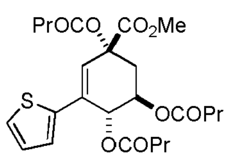 | **18** | >160 | >160 |
| 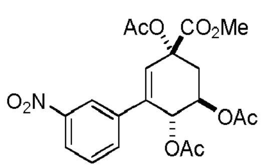 | **19** | >160 | >160 |
| 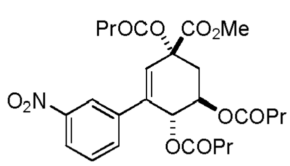 | **20** | >160 | >160 |

## Claims

1. A compound of formula **I**, its diastereoisomers, its enantiomers or its pharmaceutically acceptable salts or solvates

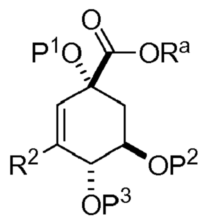

**I**

wherein,
$R^a$ is selected from the group consisting of substituted or unsubstituted alkyl, substituted or unsubstituted alkenyl, substituted or unsubstituted alkynyl, substituted or unsubstituted aryl, substituted or unsubstituted heterocyclyl, substituted or unsubstituted arylalkyl and substituted or unsubstituted heterocyclylalkyl;
each $P^1$, $P^2$ and $P^3$ is independently selected from the group consisting of hydrogen, substituted or unsubstituted alkyl, substituted or unsubstituted alkenyl, substituted or unsubstituted alkynyl, substituted or unsubstituted silyl, substituted or unsubstituted arylalkyl and -(C=O)$R^a$, wherein $R^a$ is as defined above; and
$R^2$ is selected from the group consisting of substituted or unsubstituted alkyl, substituted or unsubstituted alkenyl, substituted or unsubstituted alkynyl, substituted or unsubstituted aryl, substituted or unsubstituted heterocyclyl, substituted or unsubstituted arylalkyl and substituted or unsubstituted heterocyclylalkyl.

2. A compound according to claim 1, wherein $R^2$ is selected from the group consisting of substituted or unsubstituted aryl, substituted or unsubstituted heterocyclyl, substituted or unsubstituted arylalkyl and substituted or unsubstituted heterocyclylalkyl.

3. A compound according to claim 1 or 2, wherein $R^2$ is a group of formula **II**

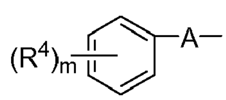

**II**

wherein
A is a bond, or is selected from a substituted or unsubstituted alkyl chain, a substituted or unsubstituted alkenyl chain, or a substituted or unsubstituted alkynyl chain;
m is 0, 1, 2, 3, 4 or 5; and
$R^4$ is selected from the group consisting of halogen, substituted or unsubstituted alkyl, substituted or unsubstituted alkenyl, substituted or unsubstituted alkynyl, substituted or unsubstituted alkoxy, substituted or unsubstituted thioalkoxy, substituted or unsubstituted aryl, substituted or unsubstituted heterocyclyl, substituted or unsubstituted arylalkyl and substituted or unsubstituted heterocyclylalkyl;
wherein $R^4$ may be in any of the free positions.

4. Compound according to claim 3, wherein $R^4$ is selected from the group consisting of halogen, $CF_3$, nitro, cyano, hydroxyl, and carboxy.

5. Compound according to claim 1 or 2, wherein $R^2$ is a group of formula **III**

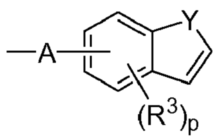

**III**

wherein
A is a bond, or is selected from a substituted or unsubstituted alkyl chain, a substituted or unsubstituted alkenyl chain, or a substituted or unsubstituted alkynyl chain;
p is 0, 1, 2, or 3;
$R^3$ is selected from the group consisting of hydrogen, substituted or unsubstituted alkyl, substituted or unsubstituted alkenyl, substituted or unsubstituted alkynyl, substituted or unsubstituted alkoxy, substituted or unsubstituted thioalkoxy, substituted or unsubstituted aryl, substituted or unsubstituted heterocyclyl, substituted or unsubstituted arylalkyl and substituted or unsubstituted heterocyclylalkyl; and
Y is selected from the group consisting of O, S, $NR^b$ and $\oplus NR^bR^c$, wherein each of $R^b$ and $R^c$ is independently selected from the group consisting of hydrogen, substituted or unsubstituted alkyl, substituted or unsubstituted alkenyl, substituted or unsubstituted alkynyl, substituted or unsubstituted aryl, substituted or unsubstituted heterocyclyl, substituted or unsubstituted arylalkyl and substituted or unsubstituted heterocyclylalkyl; or $R^b$ and $R^c$ together form a 5 or 6 membered heterocyclyc ring together with the nitrogen atom to which they are attached,
wherein A and $R^3$ may be in any of the free positions.

**6.** Compound according to claim 1 or 2, wherein $R^2$ is a group of formula **IV**

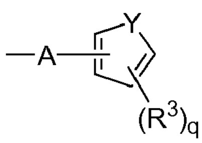

**IV**

wherein
A, Y and $R^3$ are as defined in claim 5; and
q is 0, 1, 2, or 3;
wherein A and $R^3$ may be in any of the free positions.

**7.** Compound according to any of the previous claims, wherein Ra is a $C_{1-4}$ alkyl group.

**8.** A compound of formula **I** according to claim 1, selected from the group consisting of:

isopropyl (1R, 4R, 5R)-1,4,5-trihydroxy-3(3-phenylpropyl)cyclohex-2-en-1-carboxylate;
ethyl (1R, 4R, 5R)-1,4,5-trihydroxy-3(3-phenylpropyl)cyclohex-2-en-1-carboxylate;
methyl (1R, 4R, 5R)-1,4,5-trihydroxy-3(3-phenylpropyl)cyclohex-2-en-1-carboxylate;
methyl (1R, 4R, 5R)-1,4,5-trihydroxy-3-(naphthalen-2-yl)cyclohex-2-en-1-carboxylate;
methyl (1R, 4R, 5R)-3-(benzofuran-5-yl)-1,4,5-trihydroxycyclohex-2-en-1-carboxylate;
methyl (1R, 4R, 5R)-3-(furan-2-yl)-1,4,5-trihydroxycyclohex-2-en-1-carboxylate;
methyl (1R, 4R, 5R)-3-(4-fluorophenyl)-1,4,5-trihydroxycyclohex-2-en-1-carboxylate;
methyl (1R, 4R, 5R)-1,4,5-trihydroxy-3-(3-nitrophenyl)cyclohex-2-en-1-carboxylate;
methyl (1R, 4R, 5R)-3-(benzo[b]thiophen-5-yl)-1,4,5-trihydroxycyclohex-2-en-1-carboxylate;
methyl (1R, 4R, 5R)-1,4,5-trihydroxy-3-(thien-2-yl)cyclohex-2-en-1-carboxylate;
ethyl (1R, 4R, 5R)-1,4,5-triacetoxy-3(3-phenylpropyl)cyclohex-2-en-1-carboxylate;
methyl (1R, 4R, 5R)-1,4,5-triacetoxy-3(3-phenylpropyl)cyclohex-2-en-1-carboxylate;
isopropyl (1R, 4R, 5R)-1,4,5-triacetoxy-3(3-phenylpropyl)cyclohex-2-en-1-carboxylate;
methyl (1R, 4R, 5R)-1,4,5-tributyroxy-3(3-phenylpropyl)cyclohex-2-en-1-carboxylate;
methyl (1R, 4R, 5R)-1,4,5-triacetoxy-3-(naphthalen-2-yl)cyclohex-2-en-1-carboxylate;
methyl (1R, 4R, 5R)-1,4,5-triacetoxy-3-(benzofuran-5-yl)cyclohex-2-en-1-carboxylate;

methyl (1R, 4R, 5R)-3-(benzofuran-5-yl)-1,4,5-tributyroxycyclohex-2-en-1-carboxylate;
methyl (1R, 4R, 5R)-1,4,5-triacetoxy-3-(furan-2-yl)cyclohex-2-en-1-carboxylate;
methyl (1R, 4R, 5R)-1,4,5-triburyroxy-3-(furan-2-yl)cyclohex-2-en-1-carboxylate;
methyl (1R, 4R, 5R)-1,4,5-triacetoxy-3-(benzo[b]thiophen-5-yl)cyclohex-2-en-1-carboxylate;
methyl (1R, 4R, 5R)-3-(benzo[b]thiophen-5-yl)-1,4,5-tributyroxycyclohex-2-en-1-carboxylate;
methyl (1R, 4R, 5R)-1,4,5-triacetoxy-3-(thien-2-yl)cyclohex-2-en-1-carboxylate;
methyl (1R, 4R, 5R)-1,4,5-tributyroxy-3-(naphthalen-2-yl)cyclohex-2-en-1-carboxylate;
methyl (1R, 4R, 5R)-3-(naphthalen-2-yl)-1,4,5-tripentyroxycyclohex-2-en-1-carboxylate;
methyl (1R, 4R, 5R)-1,4,5-tributyroxy-3-(thien-2-yl)cyclohex-2-en-1-carboxylate;
methyl (1R, 4R, 5R)-1,4,5-triacetoxy-3-(3-nitrophenyl)cyclohex-2-en-1-carboxylate;
methyl (1R, 4R, 5R)-1,4,5-tributyroxy-3-(3-nitrophenyl)cyclohex-2-en-1-carboxylate;
and
methyl (1R, 4R, 5R)-1,4,5-triacetoxy-3-(4-fluorophenyl)-cyclohex-2-en-1-carboxylate;
its diastereoisomers, its enantiomers or its pharmaceutically acceptable salts or solvates.

**9.** Process for the preparation of compounds of formula **I** as defined in any of claims 1 to 8, comprising the ring opening of a lactone of formula **V** in the presence of a base and an alcohol of formula $HOR^a$

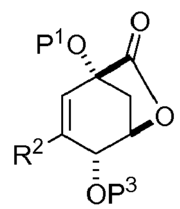

**V**

wherein $P^1$, $P^3$, $R^2$ and $R^a$ are as defined in claim 1.

**10.** Process for the preparation of compounds of formula **I** as defined in any of claims 1 to 8, comprising the esterification of an acid of formula **VI**

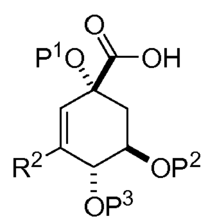

**VI**

wherein $P^1$, $P^2$, $P^3$, and $R^2$ are as defined in claim 1.

**11.** A pharmaceutical composition comprising a compound of formula **I** as defined in any of claims 1 to 8 and a pharmaceutically acceptable carier.

**12.** A compound of formula **I** as defined in anyone of claims 1 to 8, for use as a medicament.

**13.** A compound of formula **I** as defined in anyone of claims 1 to 8, for use as an antibiotic and/or antimicrobial agent.

**14.** Compound according to claim 13 for use in the treatment or prophylaxis of a disease selected from the group consisting of tuberculosis, stomach cancer, gastritis, stomach ulcers, and duodenal ulcers heartburn.

Europäisches Patentamt
European Patent Office
Office européen des brevets

# EUROPEAN SEARCH REPORT

Application Number
EP 09 38 2098

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TOSCANO MIGUEL D ET AL: "Nanomolar inhibition of type II dehydroquinase based on the enolate reaction mechanism" CHEMMEDCHEM, WILEY - VCH VERLAG., WEINHEIM, DE, vol. 2, no. 1, 1 January 2007 (2007-01-01), pages 101-112, XP002527333 ISSN: 1860-7179 * the whole document * ----- | 1-13 | INV. C07C205/55 C07C69/757 C07D307/46 C07D307/80 C07D333/72 A61K31/216 A61K31/341 A61K31/343 A61K31/381 A61P31/06 A61P31/04 A61P31/00 |
| A | SANCHEZ-SIXTO C ET AL: "Structure-based design, synthesis, and biological evaluation of inhibitors of Mycobacterium tuberculosis type II dehydroquinase" JOURNAL OF MEDICINAL CHEMISTRY, AMERICAN CHEMICAL SOCIETY, WASHINGTON, US, vol. 48, no. 15, 28 July 2005 (2005-07-28), pages 4871-4881, XP002527352 ISSN: 0022-2623 [retrieved on 2005-06-25] * the whole document * ----- | 1-13 | |
| | | | TECHNICAL FIELDS SEARCHED (IPC) |
| D,A | EP 1 647 544 A (UNIV SANTIAGO COMPOSTELA [ES]) 19 April 2006 (2006-04-19) * the whole document * ----- | 1-13 | C07C C07D |
| D,A | C. GONZALES-BELLO, L. CASTEDO: "Progress in Type II Dehydroquinase Inhibitors: From Concept to Practice" MEDICINAL RESEARCH REVIEWS, vol. 27, no. 2, 2007, pages 177-208, XP002552925 * the whole document * ----- | 1-13 | |

The present search report has been drawn up for all claims

| Place of search | Date of completion of the search | Examiner |
|---|---|---|
| Munich | 29 October 2009 | Bedel, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 09 38 2098

This annex lists the patent family members relating to the patent documents cited in the above-mentioned European search report.
The members are as contained in the European Patent Office EDP file on
The European Patent Office is in no way liable for these particulars which are merely given for the purpose of information.

29-10-2009

| Patent document cited in search report | Publication date | Patent family member(s) | Publication date |
|---|---|---|---|
| EP 1647544 A | 19-04-2006 | ES 2223284 A1 | 16-02-2005 |
| | | WO 2005009330 A2 | 03-02-2005 |
| | | US 2007185214 A1 | 09-08-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005009330 A
- EP 08382085 A


### Non-patent literature cited in the description


- **Haslam, E.** The shikimate pathway. Wiley, 1974
- Enzymology and molecular biology of the shikimate pathway. **Abell, C.** Comprehensive Natural Products Chemistry. Oxford: Pergamon, Elsevier Science Ltd, 1998, 573
- **Roberts, F.** *Nature,* 1998, vol. 393, 801
- **Roberts, C. W.** *J. Infect. Dis.,* 2002, vol. 185 (1), S25
- **McConkey, G. A. ; Pinney, J. W. ; Westhead, D. R. ; Plueckhahn, K. ; Fitzpatrick, T. B. ; Macheroux, P. ; Kappes, B.** *Trends in Parasitology,* 2004, vol. 20, 60
- **Hawkins, A. R.** *Curr. Genet.,* 1987, vol. 11, 491
- **Kleanthous, C. ; Davis, K. ; Kelly, S. M. ; Cooper, A. ; Harding, S. E. ; Price, N. C. ; Hawkins, A. R. ; Coggins, J. R.** *Biochem. J.,* 1992, vol. 282, 687
- **Gourley, D. G. ; Coggins, J. R. ; Isaacs, N. W. ; Moore, J. D. ; Charles, I. G. ; Hawkins, A. R. J.** *Mol. Biol.,* 1994, vol. 241, 488
- **Krell, T. ; Pitt, A. R. ; Coggins, J. R.** *FEBS Lett.,* 1995, vol. 360, 93
- **González-Bello, C. et al.** *Org. Biomol. Chem.,* 2003, vol. 1, 2075-2083
- **González-Bello, C. et al.** *Medicinal Research Reviews,* 2007, vol. 27 (2), 177-208
- **González-Bello, C. et al.** *ChemMedChem,* 2008, vol. 3, 756-770
- **González-Bello, C. et al.** *ChemMedChem,* 2007, vol. 2, 194-207
- **Greene, T. W. ; Wuts, P. G. M.** Protective Groups in Organic Synthesis. Wiley-Interscience, 1999
- **March, J.** Advanced Organic Chemistry; Reactions Mechanism and Structure. Wiley-Interscience, 486-7
- **March, J.** Advanced Organic Chemistry; Reactions Mechanism and Structure. Wiley-Interscience, 484-6
- **March, J.** Advanced Organic Chemistry; Reactions Mechanism and Structure. Wiley-Interscience, 490
- **González-Bello, C. et al.** *ChemMedChem.,* 2007, vol. 2, 194-207
- **González-Bello, C. et al.** *ChemMedChem.,* 2008, vol. 3, 756-770
- *J. Med. Chem.,* 2005, vol. 48, 4871-4881
- **March, J.** Advanced Organic Chemistry; Reactions Mechanism and Structure. Wiley-Interscience, 1002-7
- **March, J.** Advanced Organic Chemistry; Reactions Mechanism and Structure. Wiley-Interscience, 482-3
- **González-Bello, C.** *ChemMedChem,* 2007, vol. 2, 194-207
- **González-Bello, C.** *ChemMedChem,* 2008, vol. 3, 756-770
- **González-Bello, C.** *J. Med. Chem.,* 2005, vol. 48, 4871-4881
- **Palomino, J. C. et al.** *Antimicrob. Agents Chemother.,* 2002, vol. 46, 2720-2722